# EUROPEAN PATENT APPLICATION

(11) **EP 4 488 277 A1**
(43) Date of publication of application: **08.01.2025**
(21) Application number: 23306128.2
(22) Date of filing: 04.07.2023
(51) Int. Cl.: C07D 471/08, C07B 59/00, A61K 51/04

(54) **NOVEL BISPIDINE-BASED METAL CHELATING LIGANDS DISPLAYING GOOD RELAXIVITY**

(71) Applicant: Université de Strasbourg, 67000 Strasbourg (FR); Centre National de la Recherche Scientifique, 75016 Paris (FR)
(72) Inventor: NONAT, Aline, 67201 Eckbolsheim (FR); CHARBONNIERE, Loïc, 67720 Weyersheim (FR); SY, Maryame, 94600 Choisy-le-Roi (FR); CHARPENTIER, Cyrille, 67000 Strasbourg (FR); JAKAB TOTH, Eva, 45160 Olivet (FR)
(74) Representative: IPAZ

(57) **Abstract**

The present invention relates to a novel bispidine-based metal chelating ligand comprising several bispidol compounds covalently linked to each other via a specific linker, each bispidol compound comprising at the N7-position of the bispidine scaffold at least one substituted ethanoic acid or methyl phosphonic acid moiety, said bispidine-based metal chelating ligand being able to form metallic complexes having good properties in terms of relaxivity, to a complex comprising several metal ions complexed with said bispidine-based metal chelating ligand, and to the use of said bispidine-based metal chelating ligand in the field of medical imaging or therapy, and more specifically as MRI (magnetic resonance imaging) contrast agents and/or nuclear imaging agents for PET (positron emission tomography) or SPECT (single photon emission tomography).

## Description

The present invention relates to a novel bispidine-based metal chelating ligand comprising several bispidol compounds covalently linked to each other via a specific linker, each bispidol compound comprising at the N7-position of the bispidine scaffold at least one substituted ethanoic acid or methyl phosphonic acid moiety, said bispidine-based metal chelating ligand being able to form metallic complexes having good properties in terms of relaxivity, to a complex comprising several metal ions complexed with said bispidine-based metal chelating ligand. The present invention relates also to the use of said bispidine-based metal chelating ligand in the field of medical imaging or therapy, and more specifically as MRI (magnetic resonance imaging) contrast agents and/or nuclear imaging agents for PET (positron emission tomography) or SPECT (single photon emission tomography).

Gadolinium-complexes have been used in millions of human examinations with magnetic resonance imaging (MRI) and are considered among the safest diagnostic drugs. The following gadolinium-based contrast agents (GBCAs) have been approved for clinical use: gadopentetate dimeglumine or [Gd(OH₂)(dtpa)]²⁻ (Magnevist^{®}, compound "a" in Scheme 1 below), gadoteridol (ProHance^{®}), gadodiamide or [Gd(OH₂)(dtpa-bma)] (Omniscan^{®}, compound "b" in Scheme 1 below), gadoterate meglumine or [Gd(OH₂)(dota)]⁻ (Dotarem^{®}, compound "c" in Scheme 1 below), gadobutrol or [Gd(OH₂)(do3a-butrol)] (Gadovist^{®}, compound "d" in Scheme 1 below), gadoversetamide (OptiMARK^{®}), gadoxetic acid (Primovist^{®}), gadobenate dimeglumine or [Gd(OH₂)(bopta)]²⁻ (MultiHance^{®}, compound e in Scheme 1 below), and gadofosveset trisodium (Vasovist^{®}/Ablavar^{®}).

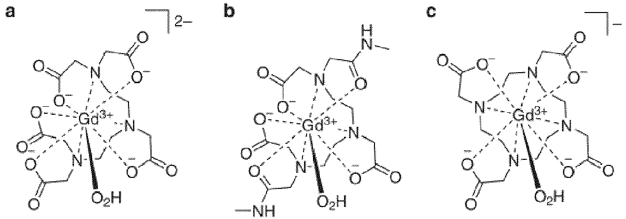

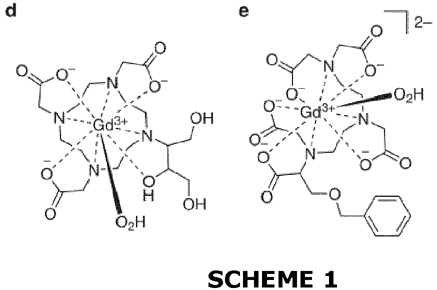

However, the recent emergence of nephrogenic systemic fibrosis and its causal link to Gd exposure, as well as the evidence on brain and bone accumulation of Gd have alerted the medical community. Nephrogenic systemic fibrosis (NSF) is a rare and serious syndrome that is associated with the exposure to GBCAs in patients with chronic kidney disease. NSF involves fibrotic changes in the skin and many organs and can be a lethal disease.

Therefore, in 2010, the U.S. Food and Drug Administration (FDA) published revised labelling recommendations for four linear GBCAs which have been principally implicated in NSF, including gadodiamide (Omniscan^{®}), gadobenate dimeglumine (MultiHance^{®}), gadopentetate dimeglumine (Magnevist^{®}), and gadoversetamide (OptiMARK^{®}). In 2017, the European Medicines Agency has decided to stop marketing linear GBCAs contrast agents.

Furthermore, the negative outcome of using high quantities of Gd(III) based contrast agents, e.g. gadolinium accumulating in surface waters and coming from clinical waste waters, also arises an increasing environmental problem.

In this context, providing novel Gd(III) based contrast agents that can be administered in lower quantities and/or that are more stable as well as replacing Gd(III) with more biocompatible, safer paramagnetic metal ions have become major objectives.

Indeed, due to the low sensitivity of MRI as an imaging technique, large quantities of a contrast agent, often on the gram scale, must be injected into the patient to obtain useful images. The ability to reduce the quantity of GBCAs required is highly desirable, especially when considering the toxicity problems discussed above. One way in which the amount of contrast agent required can be reduced is to enhance its relaxivity. Relaxivity is the ability of the metal chelate to relax water protons, and is defined as the change in the relaxation rate of water divided by the millimolar concentration of the chelate. High relaxivities are indicative of more effective agents. All the commercially available GBCAs are very similar to each other in terms of relaxivity. As an example, Dotarem^{®} displays a relaxivity *r₁* of 3.7 mM⁻¹.s⁻¹ at pH of 7.4, 60 MHz, and 25°C.

Another way to reduce or avoid the risks of health problems associated with Gd³⁺ release is to provide stable GBCAs. Gd³⁺ release by GBCAs can be characterised by thermodynamic stability and kinetic inertness. Thermodynamic stability refers to the Gibbs free energy involved in the complexation reaction and is defined by the stability constant log *K* (also called log *K*_{GdL}, log *K*ₜₕₑᵣₘ and log *K*ₛₜ). Kinetic inertness refers to complex dissociation rate and is mainly reported as *t*_{1/2}, where *t*_{1/2} is defined as the time required for half of the GBCA's dissociation.

Research is also focused on providing alternative gadolinium(III)-free metal complexes. For example, US2020/157099 A1 describes Mn(II), Fe(II), Fe(III), Co(II) and Ni(II) ion macrocycle-based complexes to apply as MRI contrast agents and ⁵²Mn-based PET diagnostic. The compound responding to the following formula: 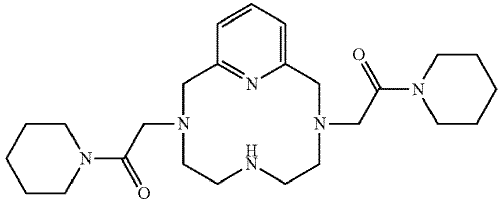 is complexed with Mn(II) so as to form a Mn(III) macrocycle metal complexe [Mn(tPC2AM*^{Pyp}*)]²⁺ having a relaxivity *r₁* of 4.90 mM⁻¹.s⁻¹ at pH of 7.4 and 25°C and an half-life t_{1/2} of complex dissociation calculated at physiologic pH of 352 hours. However, the kinetic inertness of the complex is not completely satisfactory.

Mangafodipir trisodium (Mn-DPDP), a contrast agent free of Gd(III), was marketed in the 1990's under the tradename Teslascan^{®} comprising Mn(II) ion as the central paramagnetic ion. However, it was withdrawn from the US market in 2003 and from the EU market in 2010 due to low sales, poor clinical performance, and concerns over toxicity.

Accordingly, there is a need of novel metal chelating ligands able to form complexes with other metals than gadolinium(III) and which have improved relaxivity while guaranteeing good kinetic inertness and good solubility.

Thus, the aim of the present invention is to overcome the drawbacks of the cited prior art, and more particularly, to provide novel metal chelating ligands having good relaxation properties, low toxicity and/or which can be easily eliminated by renal route, water solubility, good kinetic inertness and low dissociation rate.

A first object of the present invention is thus a bispidine-based metal chelating ligand responding to the following formula (I):

L-(R)ₙ (I)

wherein n represents an integer such as n ≥ 2, and R groups represent, independently from each other,
   - a group R¹-(T¹)ᵣ-CH₂^{∗}-, in which T¹ represents a C₁-C₁₀ alkylene group, and r represents an integer 0 or 1; and/or
   - a group responding to the following formula (II): 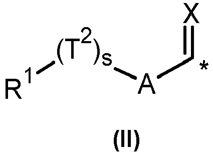 in which:
      - T² represents a C₁-C₁₀ alkylene group,
      - A represents -CH₂- or -NH-,
      - X represents an oxygen atom, a sulfur atom, or a NH group, and
      - s represents an integer 0 or 1 when A represents -CH₂- and s represents an integer 1 when A represents -NH-, and
wherein * indicates the point of attachment of said group R with L,
wherein R¹ is a bispidol group of the following formula (III): 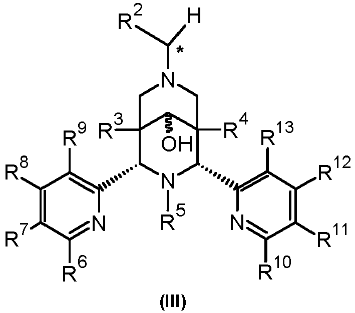 in which:
   - R² represents a PO₃H₂ group or a CO₂H group,
   - R³ and R⁴, which may be identical or different, represent a CO₂H group or a CH₂OH group,
   - R⁵ represents a hydrogen atom, an alkyl group, or a group of formula (IV): -T³-CO₂H (IV) where T³ represents a C₁-C₅ alkylene group,
   - R⁶, R⁷, R⁸, R⁹, R¹⁰, R¹¹, R¹², and R¹³, independently from each other, represent a hydrogen atom, an OH group, an ether group OR¹⁴ where R¹⁴ is an alkyl group, a CO₂H group, or a CONHR¹⁵ group, where R¹⁵ represents an alkyl group,
   - when r, respectively s, represents an integer 1, * indicates the point of attachment of said group R¹ with T¹, respectively with T², and when r, respectively s, represents an integer 0, ^{∗} indicates the point of attachment of said group R¹ with -CH₂-, respectively with -A-;
wherein L is at least a divalent linker selected from:
   - a single bond,
   - a guadinine group,
   - an urea group,
   - a thiourea group,
   - a C₅-C₁₈ aryl group,
   - a C₂-C₁₈ heteroaryl group,
   - a C₂-C₁₀ alkyl,
   - a group responding to formula (V):

      *NH-D(NH*)t-NH* (V)

      in which D is selected from an aryl group, an heteroaryl group, an alkyl group, a 3-cyclobutene-1,2-dione group, a polyethylene group, and a polyaminoacid group, and t is an integer ranging from 0 to 6,
   - a group responding to the following formula (VI):

      ^{∗}NH-(CR¹⁶R¹⁷)ₘ₁-[NR²⁰-(CR¹⁸R¹⁹)_{q1}]ₚ₁-NH^{∗} (VI)

      in which:
      - R¹⁶ and R¹⁷ represent, independently from each other, independently at each occurrence m1, a hydrogen atom or a C₁-C₅ alkyl group,
      - m1 is an integer ranging from 2 to 5,
      - R¹⁸ and R¹⁹ represent, independently from each other, independently at each occurrence q1, independently at each occurrence p1, a hydrogen atom or a C₁-C₅ alkyl group,
      - R²⁰ represents, independently at each occurrence p1, a hydrogen atom, a C₁-C₅ alkyl group, a group -T⁴-NH^{∗} in which T⁴ represents a C₂-C₅ alkylene group, or a single bond*,
      - q1 represents, independently at each occurrence p1, an integer ranging from 2 to 5, and
      - p1 is an integer ranging from 0 to 4,
   - a group responding to the following formula (VII):

      ^{∗}E-NH-(CR¹⁶R¹⁷)ₘ₁-[NR²¹-(CR¹⁸R¹⁹)_{q1}]ₚ₁-NH-E^{∗} (VII)

      in which:
      - R¹⁶, R¹⁷, R¹⁸, and R¹⁹ are as defined above,
      - m1, q1, and p1 are as defined above,
      - R²¹ represents, independently at each occurrence p1, a hydrogen atom, a C₁-C₅ alkyl group, a group -T⁵-NH-E^{∗} in which T⁵ represents a C₂-C₅ alkylene group, or a single bond*,
      - E is a 3-amino-, or 4-amino-3-cyclobutene-1,2-dione group, or a C₁-C₅ alkyl group,
   - a group responding to the following formula (VIII):

      ^{∗}(CR²²R²³)ₘ₂-NR²⁸-[(CR²⁴R²⁵)_{q2}-NR²⁹]ₚ₂-(CR²⁶R²⁷)ₘ₃^{∗} (VIII)

      in which:
      - R²² and R²³ represent, independently from each other, independently at each occurrence m2, a hydrogen atom or a C₁-C₅ alkyl group,
      - m2 is an integer ranging from 1 to 5,
      - R²⁴ and R²⁵ represent, independently from each other, independently at each occurrence q2, independently at each occurrence p2, a hydrogen atom or a C₁-C₅ alkyl group,
      - R²⁶ and R²⁷ represent, independently from each other, independently at each occurrence m3, a hydrogen atom or a C₁-C₅ alkyl group,
      - m3 is an integer ranging from 1 to 5,
      - R²⁸ represents a hydrogen atom, a C₁-C₅ alkyl group, a C₁-C₅ alkylene group*, or a single bond*,
      - R²⁹ represents, independently at each occurrence p2, a hydrogen atom, a C₁-C₅ alkyl group, a C₁-C₅ alkylene group*, a single bond*, or a - [(CR²⁴R²⁵)_{q2}-NR²⁹]ₚ₂-(CR²⁶R²⁷)ₘ₃^{∗} group,
      - q2 represents, independently at each occurrence p2, an integer ranging from 2 to 5, and
      - p2 is an integer ranging from 0 to 4,
   - a group responding to the following formula (IX):

      ^{∗}(CR³⁰R³¹)ₘ₄-NR³⁴-[(CH₂-CH₂-O)_{q4}-CH₂-CH₂-NR³⁵]ₚ₄-(CR³²R³³)ₘ₅^{∗} (IX)

      in which:
      - R³⁰ and R³¹ represent, independently from each other, independently at each occurrence m4, a hydrogen atom or a C₁-C₅ alkyl group,
      - m4 is an integer ranging from 1 to 5,
      - R³² and R³³ represent, independently from each other, independently at each occurrence m5, a hydrogen atom or a C₁-C₅ alkyl group,
      - m5 is an integer ranging from 1 to 5,
      - R³⁴ represents a hydrogen atom, a C₁-C₅ alkyl group, a C₁-C₅ alkylene group*, or a single bond*,
      - R³⁵ represents, independently at each occurrence p4, a hydrogen atom, a C₁-C₅ alkyl group, a C₁-C₅ alkylene group*, or a single bond*,
      - q4 represents, independently at each occurrence p4, an integer ranging from 1 to 5, and
      - p4 is an integer ranging from 0 to 4,
      wherein * in formulae (V) to (IX) indicates the possible points of attachment of said linker L with groups R,
   - a polyazacycloalkane saturated group in which at least two -N-functions represent points of attachment of said linker L with groups R,
   - a polyaminoacid group in which at least two functions represent points of attachment of said linker L with groups R, said function being a carbonyl function (derived from the carboxylic acid function) a thiol function, or an amine function,
   - a polyamidoamine group in which at least two -NH- functions represent points of attachment of said linker L with groups R.

Thus, the ligand of the invention can lead to a metal complex having good relaxation properties, low toxicity and/or which can be easily eliminated by renal route, water solubility, good improved kinetic inertness and low dissociation rate.

Bispidines are chelators based on a 3,7-diazabicyclo[3.3.1]nonane scaffold. The bispidine scaffold is formed by two fused cyclohexylamine rings.

More particularly, the bispidol groups R¹ of formula (III) present in the metal chelating ligand (I) of the present invention have a so-called 2,4-disubstituted bispidol core and their IUPAC numbering is presented as follows:

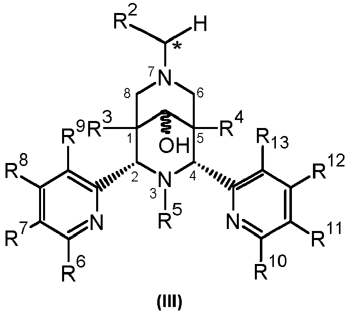

Said bispidol group R¹ of formula (III) is expected to coordinate at least in a pentadentate manner, involving two pyridine and two bispidine nitrogens as well as the methylene carboxylate moiety or the methylene phosphonate moiety at the N7 position. Additionally the ligand of the present invention comprises several bispidol groups R¹ of formula (III) linked to each other through appropriate chemical groups linked to the methylene carboxylate or phosphonate moiety -CH^{∗}-R², i.e. appropriate L linkers and appropriate T¹, T², A, X groups within the R groups leading to improved relaxivity with similar coordination and hence kinetic inertness or dissociation rate.

The bispidine-based metal chelating ligands of the present invention are highly preorganized ligands that can accommodate several metal ions with cis-octahedral, square-pyramidal, or pentagonal geometries. The bispidine-based metal chelating ligands of the invention form thermodynamically very stable metal complexes with transition-metal ions, and in particular with Mn(II), which show high relaxivity. Modification of the coordinating pendant arms can be used to tune the ligand denticity as well as all electronic, thermodynamic, and kinetic parameters such as the ligand field, the metal selectivity, and the stability constants. Such properties are very appealing for applications in diagnosis as chelators for metals other than gadolinium, and more specifically for manganese and copper metals, as well as radiometals such as ⁶⁴Cu, ⁵²Mn and ⁶⁷Ga.

In the present invention, the term "alkyl group" refers to a saturated linear, branched, or cyclic (branched or unbranched) hydrocarbon chain, generally comprising from 1 to 12 carbon atoms, preferably from 1 to 8 carbon atoms, and more preferably from 1 to 4 carbon atoms. In the present invention, alkyl groups may be monovalent or polyvalent (*i.e*., alkylene groups are encompassed in "alkyl" definition).

In the present invention, the term "alkylene group" refers to a divalent alkyl group.

In the present invention, the term "aryl group" refers to a cyclic, polyunsaturated, aromatic hydrocarbyl group comprising at least one aromatic ring and comprising from 5 to 20 carbon atoms, preferably from 5 to 18 carbon atoms. Aryl groups may have a single ring or multiple aromatic rings fused together or linked covalently.

In the present invention, the terms "Cx-Cy" or "(Cx-Cy)" preceding the name of a group means that the group comprises from x to y carbon atoms, in accordance to common terminology in the chemistry field.

In the present invention, the term "heteroaryl" refers to cyclic, polyunsaturated, aromatic hydrocarbyl group comprising at least one aromatic ring and comprising from 2 to 20 carbon atoms, preferably from 2 to 18 carbon atoms, having one or two rings which are fused together or linked covalently, wherein one or more carbon atoms in one or more of these rings is replaced by oxygen, nitrogen and/or sulfur atoms. The nitrogen and sulfur heteroatoms may optionally be oxidized and the nitrogen heteroatoms may optionally be quaternized.

### The bispidol group R¹ of formula (III)

The bispidol group R¹ of formula (III) is either covalently linked to T¹ in the group R¹-(T¹)ᵣ-CH₂^{∗}- when r represents an integer 1, or covalently linked to CH₂ in the group R¹-(T¹)ᵣ-CH₂^{∗}- when r represents an integer 0.

The bispidol group R¹ of formula (III) is either covalently linked to T² in the group R¹-(T²)ₛ-A-C(=X)^{∗}- when s represents an integer 1, or covalently linked to A in the group R¹-(T²)ₛ-A-C(=X)^{∗}- when s represents an integer 0.

### The R² group

R² represents a PO₃H₂ group or a CO₂H group, and preferably a CO₂H group.

### The R³ and R⁴ groups

R³ and R⁴, which may be identical or different, represent a CO₂H group or a CH₂OH group.

R³ and R⁴ are preferably identical.

In one preferred embodiment, R³ and R⁴ represent CO₂H groups.

### The R⁵ group

R⁵ represents a hydrogen atom, an alkyl group, or a group of formula (IV): -T³-CO₂H (IV) where T³ represents a C₁-C₅ alkylene group, preferably an alkyl group or a group of formula (IV): -T³-CO₂H (IV) where T³ represents a C₁-C₅ alkylene group, and more preferably an alkyl group.

The alkyl group as R⁵ can be a linear or a branched alkyl group, and preferably a linear alkyl group.

In the present invention, the term "linear" means an unsubstituted or unbranched.

The alkyl group as R⁵ is preferably a C₁-C₅ alkyl group, and more preferably a methyl group.

In one particularly preferred embodiment, R⁵ is a linear C₁-C₅ alkyl group.

In the group of formula (IV): -T³-CO₂H (IV), T³ represents a C₁-C₅ alkylene group.

In the present invention, the term "alkylene" means an alkyl group which is divalent. The term "alkyl" means a saturated aliphatic hydrocarbon radical.

In the present invention, the term "aliphatic hydrocarbon radical" means a hydrocarbonated chain and the term "aliphatic" is opposed to "aromatic".

The C₁-C₅ alkylene group as T³ can be a linear or a branched alkylene group, and preferably a linear alkylene group.

T³ preferably represents a C₁-C₃ alkylene group.

### The R⁶, R⁷, R⁸, R⁹, R¹⁰, R¹¹, R¹², and R¹³ groups

R⁶, R⁷, R⁸, R⁹, R¹⁰, R¹¹, R¹², and R¹³, independently from each other, represent a hydrogen atom, an OH group, an ether group OR¹⁴ where R¹⁴ is an alkyl group, a CO₂H group, or a CONHR¹⁵ group, where R¹⁵ represents an alkyl group.

The alkyl group as R¹⁴ can be a linear or a branched alkyl group, and preferably a linear alkyl group.

The alkyl group as R¹⁴ is preferably a C₁-C₁₀ alkyl group, and more preferably a C₁-C₅ alkyl group, and even more preferably a methyl or ethyl group.

The alkyl group as R¹⁵ can be a linear, branched, or cyclic alkyl group, and preferably a linear or cyclic alkyl group.

The alkyl group as R¹⁵ is preferably a C₁-C₂₀ alkyl group, more preferably a C₁-C₁₂ alkyl group, and even more preferably a C₁-C₆ alkyl group.

In one preferred embodiment, at least R⁷ and R¹¹, and/or R⁹ and R¹³ represent hydrogen atoms. In other words, the pyridine rings can be substituted in 4 and/or 6 positions (i.e. at least R⁶ and R¹⁰, and/or R⁸ and R¹² are different from hydrogen atoms).

In one particularly preferred embodiment, R⁶, R⁷, R⁸, R⁹, R¹⁰, R¹¹, R¹², and R¹³ groups represent hydrogen atoms.

In one preferred embodiment, which can be advantageously combined with the preceding preferred embodiments, R⁶ and R¹⁰ are identical, R⁷ and R¹¹ are identical, R⁸ and R¹² are identical, and/or R⁹ and R¹³ are identical, and more preferably R⁶ and R¹⁰ are identical, R⁷ and R¹¹ are identical, R⁸ and R¹² are identical, and R⁹ and R¹³ are identical.

### The R group

In the ligand of the present invention, the linker L is covalently linked or bounded to at least two groups R (n ≥ 2) (L is at least a divalent linker).

In one preferred embodiment, 2 ≤ n ≤ 8, preferably 2 ≤ n ≤ 6, and more preferably 2 ≤ n ≤ 4.

A group R is preferably linked to the linker L through (via) a function selected from an amide function (-NH(C=O)- or -(C=O)NH-), an urea function (-NH(C=O)NH-), a thiourea (-NH(C=S)NH-), a guanidine function (-NH(C=NH)NH-), or an amine function (-NH-). In that embodiment, the function can be provided either by the linker L, the group R or both the linker and the group R.

The R groups are either groups R¹-(T¹)ᵣ-CH₂^{∗}-, groups of formula (II) R¹-(T²)ₛ-A-C(=X)^{∗}-, or mixture thereof.

The group R¹-(T¹)ᵣ-CH₂^{∗}-
r represents an integer 0 (i.e. T¹ absent) or 1 (i.e. T¹ present).

The C₁-C₁₀ alkylene group as T¹ can be a linear or a branched alkylene group, and preferably a linear alkylene group.

T¹ preferably represents a C₁-C₈ alkylene group, and more preferably a C₁-C₄ alkylene group.

In one advantageous embodiment of the present invention, the R group R¹-(T¹)ᵣ-CH₂^{∗}- is covalently linked to L via an -NH- function, a guanidine function or an urea function. In the former embodiment, the linker L may be a group responding to formula (V) and in the latter embodiments, the linker L may be a guanidine group and a urea group respectively.

The group of formula (II) R¹-(T²)ₛ-A-C(=X)^{∗}-
s represents an integer 0 (i.e. T² absent) or 1 (i.e. T² present).

T² represents a C₁-C₁₀ alkylene group.

The C₁-C₁₀ alkylene group can be a branched or a linear alkylene group, and preferably a linear alkylene group.

T² preferably represents a C₁-C₈ alkylene group, more preferably a C₁-C₆ alkylene group.

The following embodiments for the group of formula (III) are particularly advantageous:
- A represents -NH- and X represents an oxygen atom,
- A represents -NH- and X represents a sulfur atom,
- A represents -NH- and X represents a NH group,
- A represents -CH₂-, and X represents an oxygen atom.

In one advantageous embodiment of the present invention, the R group R¹-(T²)ₛ-A-C(=X)^{∗}- is covalently linked to L via an amide function (-NH(C=O)- or -(C=O)NH-), a urea (-NH(C=O)NH-) or an thiourea function (-NH(C=S)NH-). In that embodiment, the linker L may be a C₅-C₁₈ aryl group, a C₂-C₁₈ heteroaryl group, a C₂-C₁₀ alkyl group, a polyazacycloalkane saturated group, a polyaminoacid group, or any group responding to formula (V), (VI), (VII), (VIII), (IX) as defined below.

### The linker L

The linker L is at least a divalent linker and enables covalent linking of groups R to each other via said linker L.

Since L is at least a divalent linker, it comprises at least two points of attachment (mentioned by the asterisk *) so as to covalently link/bind said linker L to said groups R. In the present invention, when the linker L comprises more than two possible points of attachments of said linker L with groups R, the possible points of attachments in excess of two can either be used as points of attachment of said linker L with groups R or be attached to an hydrogen atom.

### The linker L can be a single bond.

In that embodiment, groups R are directly bounded together. For example, a group R responding to formula R¹-(T¹)ᵣ-CH₂^{∗}- is attached to another group R responding to formula R¹-(T¹)ᵣ-CH₂^{∗}- so as to form the ligand R¹-(T¹)ᵣ-CH₂-CH₂-(T¹)ᵣ-R¹, or a group R responding to formula R¹-(T¹)ᵣ-CH₂^{∗}- is attached to a group R responding to formula (II): R¹-(T²)ₛ-A-C(=X)^{∗}- so as to form the ligand R¹-(T¹)ᵣ-CH₂-C(=X)-A-(T²)ₛ-R¹; or a group R responding to formula (II): R¹-(T²)ₛ-A-C(=X)^{∗}- is attached to another group R responding to formula (II): R¹-(T²)ₛ-A-C(=X)^{∗}- so as to form the ligand R¹-(T²)ₛ-A-C(=X)-C(=X)-A-(T²)ₛ-R¹.

In that embodiment, preferred examples of ligands are the followings:
^{∗} R¹-(T¹)ᵣ-CH₂-C(=O)-A-(T²)ₛ-R¹,
^{∗} R¹-(T²)ₛ-A-C(=O)-C(=O)-A-(T²)ₛ-R¹.

### The linker L can be a quadinine group.

This embodiment is particularly advantageous when groups R respond to formula R¹-(T¹)ᵣ-CH₂^{∗}-.

### The linker L can be a urea group.

This embodiment is particularly advantageous when groups R respond to formula R¹-(T¹)ᵣ-CH₂^{∗}-.

### The linker L can be a thiourea group.

This embodiment is particularly advantageous when groups R respond to formula R¹-(T¹)ᵣ-CH₂^{∗}-.

### The linker L can be a C₅-C₁₈ aryl group.

In the present invention, the term "C₅-C₁₈ aryl" refers to aromatic rings or aromatic ring systems comprising from 5 to 18 carbon atoms, having one or two rings which are fused together or linked covalently, wherein at least one ring is aromatic.

The C₅-C₁₈ aryl group can be selected from a phenyl group, an anthracene group, a fluorene group and a naphthyl group, and preferably a phenyl group.

The C₅-C₁₈ aryl group can be substituted with one or more W substituents, preferably selected from hydroxyl, ether, primary and secondary amine, carboxylic acid, amide, nitro, and thiol substituents.

The C₅-C₁₈ aryl group is preferably a C₅-C₁₂ aryl group.

### The linker L can be a C₂-C₁₈ heteroaryl group.

In the present invention, the term "C₂-C₁₈ heteroaryl" refers to aromatic rings or aromatic ring systems comprising from 2 to 18 carbon atoms, having one or two rings which are fused together or linked covalently, wherein at least one ring is aromatic, and wherein one or more carbon atoms in one or more of these rings is replaced by heteroatom(s), preferably selected from an oxygen atom, a nitrogen atom, a sulfur atom, and mixture thereof.

The C₂-C₁₈ heteroaryl group can be selected from pyridine, diazine, pyrimidine, quinoline, indole, indazole, furan, oxazoline, pyrrole, pyrazole, pyrazine, pyridazine, cinnoline, phthalazine, quinazoline, quinaxoline, triazine, triazole, thiophene, porphyrine, aryl-based cyanine, and other aryl-based dyes, and preferably 1,3,5-triazine group and 1,2,3-triazole group.

The C₂-C₁₈ heteroaryl group can be substituted with one or more W substituents, preferably selected from hydroxyl, ether, primary and secondary amine, carboxylic acid, amide, nitro, and thiol substituents.

The C₂-C₁₈ heteroaryl group is preferably a C₂-C₁₂ heteroaryl group.

### The linker L can be a C₂-C₁₀ alkyl group.

The C₂-C₁₀ alkyl group as L can be a linear, branched or cyclic alkyl group.

The C₂-C₁₀ alkyl group is preferably a C₂-C₆ alkyl group, and even more preferably a C₂-C₄ alkyl group.

The C₂-C₁₀ alkyl group can be substituted with one or more groups selected from hydroxyl group, aminoacid group, and mixture thereof.

The aminoacid group may be selected from lysine, ornithine, arginine, histidine, aspartic acid, glutamic acid, tryptophan, and cysteine.

Examples of C₂-C₁₀ alkyl groups are the followings: 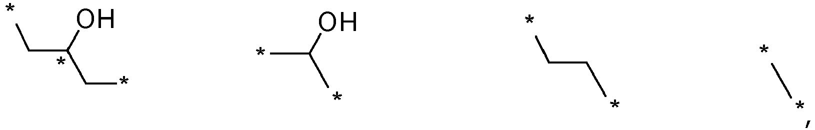 wherein * represent the points of attachment of the linker L to the group R.

### The linker L can be a group responding to formula (V):

*NH-D(NH*)t-NH* (V)

in which D is selected from an aryl group, an heteroaryl group, an alkyl group, a 3-cyclobutene-1,2-dione group, a polyethylene group, and a polyaminoacid group, and t is an integer ranging from 0 to 6, preferably t is an integer ranging from 0 to 4, and more preferably t is an integer ranging from 0 to 2.

In formula (V), * indicates the possible points of attachment of said linker L with groups R.

The aryl group as group D can be a C₅-C₁₈ aryl group, and preferably a C₅-C₁₂ aryl group.

The aryl group can be selected from a phenyl group and a naphthyl group, and preferably a phenyl group.

The aryl group can be substituted with one or more W substituents, preferably selected from hydroxyl, ether, primary and secondary amine, carboxylic acid, amide, nitro, and thiol substituents.

The heteroaryl as group D can be a C₂-C₁₈ heteroaryl group, and preferably a C₂-C₁₂ heteroaryl group.

The heteroaryl group can be selected from pyridine, diazine, pyrimidine, quinoline, indole, indazole, furan, oxazoline, pyrrole, pyrazole, pyrazine, pyridazine, cinnoline, phthalazine, quinazoline, quinaxoline, triazine, triazole, thiophene, porphyrine, aryl-based cyanine, and other aryl-based dyes, and preferably 1,3,5-triazine group and 1,2,3-triazole group.

The heteroaryl group can be substituted with one or more W substituents, preferably selected from hydroxyl, ether, primary and secondary amine, carboxylic acid, amide, nitro, and thiol substituents.

The alkyl group as group D can be a C₂-C₁₆ alkyl group, and preferably a C₂-C₁₀ alkyl group.

The alkyl group can be a linear, branched or cyclic alkyl group, and preferably a linear or cyclic alkyl group.

The alkyl group can be selected from cyclooctyl, cycloheptyl, cyclohexyl, cyclopentyl, adamantane, bicyclo[2.2.1]heptane, ethyl, and preferably cyclohexyl and adamantane.

The polyethylene group as group D can comprise several branches of--OCH₂CH₂- moities. In a particular embodiment, the polyethylene group comprises from 5 to 25 carbon atoms, and preferably from 7 to 19 carbon atoms.

The polyaminoacid group as group D comprises at least two amine functions that represent in formula (V) possible points of attachment of said linker L with groups R.

### The polyamino acid group can be a polylysine group.

In one preferred embodiment, the linker L responds to any one of the following formula (V): 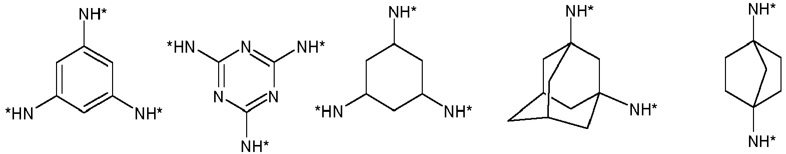 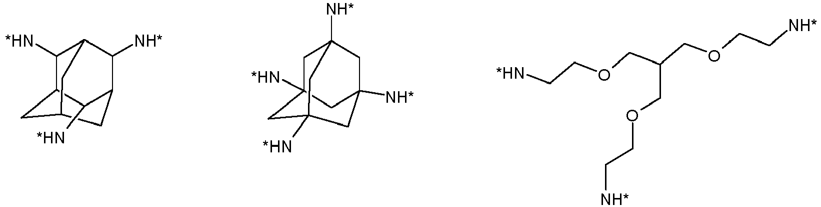 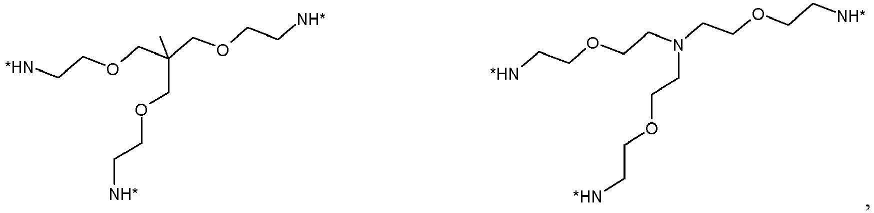 wherein * represent the possible points of attachment of the linker L to the group R.

### The linker L can be a group responding to the following formula (VI):

^{∗}NH-(CR¹⁶R¹⁷)ₘ₁-[NR²⁰-(CR¹⁸R¹⁹)_{q1}]ₚ₁-NH^{∗} (VI)

in which:
- R¹⁶ and R¹⁷ represent, independently from each other, independently at each occurrence m1, a hydrogen atom or a C₁-C₅ alkyl group,
- m1 is an integer ranging from 2 to 5, preferably ranging from 2 to 4,
- R¹⁸ and R¹⁹ represent, independently from each other, independently at each occurrence q1, independently at each occurrence p1, a hydrogen atom or a C₁-C₅ alkyl group,
- R²⁰ represents, independently at each occurrence p1, a hydrogen atom, a C₁-C₅ alkyl group, a group -T⁴-NH^{∗}- in which T⁴ represents a C₂-C₅ alkylene group, or a single bond*,
- q1 represents, independently at each occurrence p1, an integer ranging from 2 to 5, and
- p1 is an integer ranging from 0 to 4, and preferably p1 is an integer ranging from 1 to 4.

In formula (VI), * indicates the possible points of attachment of said linker L with groups R.

### R¹⁶, R¹⁷, R¹⁸, R¹⁹ and R²⁰ groups

The C₁-C₅ alkyl group as R¹⁶ group (respectively as R¹⁷, R¹⁸, R¹⁹ or R²⁰ group) can be a linear alkyl group or a branched alkyl group, and preferably a linear alkyl group.

The C₁-C₅ alkyl group is preferably a C₁-C₃ alkyl group, and more preferably a methyl or ethyl group.

The C₂-C₅ alkylene group as T⁴ can be a linear or branched alkylene group, and preferably a linear alkylene group.

The C₂-C₅ alkylene group as T⁴ is preferably a C₂-C₄ alkylene group.

When R²⁰ is a single bond, the nitrogen to which R²⁰ is attached is directly linked to a group R.

In one preferred embodiment, R²⁰ represents, independently at each occurrence p1, a hydrogen atom or a group -T⁴-NH^{∗}-.

At least one of R¹⁶ and R¹⁷ groups is preferably a hydrogen atom, both R¹⁶ and R¹⁷ groups are more preferably hydrogen atoms.

For all occurrences m1, the groups CR¹⁶R¹⁷ are preferably identical.

At least one of R¹⁸ and R¹⁹ groups is preferably a hydrogen atom, both R¹⁸ and R¹⁹ groups are more preferably hydrogen atoms.

For all occurrences q1, the groups CR¹⁸R¹⁹ are preferably identical.

In the linker L of formula (VI), there may be alternation of a radical - [NR²⁰-(CR¹⁸R¹⁹)_{q1}] where R²⁰ represents a hydrogen atom, and of a radical - [NR²⁰-(CR¹⁸R¹⁹)_{q1}] where R²⁰ represents a group -T⁴-NH^{∗}-.

In one particular preferred embodiment, the linker L responds to the any one of the following formula (VI): 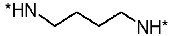 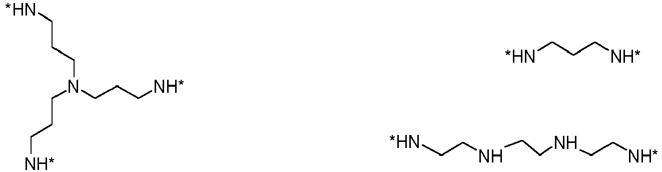 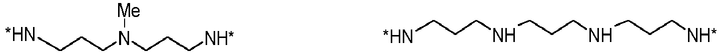 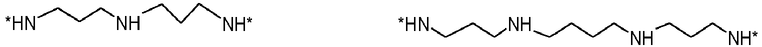 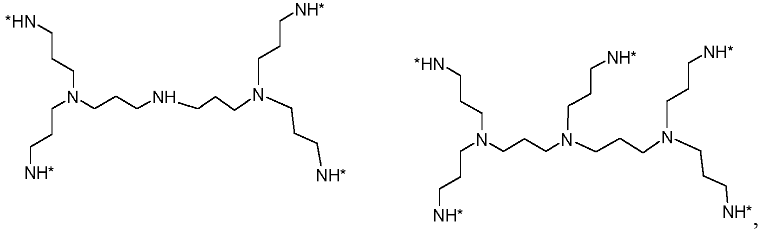 wherein * represent the possible points of attachment of the linker L to the group R.

### The linker L can be a group responding to the following formula (VII):

^{∗}E-NH-(CR¹⁶R¹⁷)ₘ₁-[NR²¹-(CR¹⁸R¹⁹)_{q1}]ₚ₁-NH-E^{∗} (VII)

in which:
- R¹⁶, R¹⁷, R¹⁸, and R¹⁹ are as defined above,
- m1, q1, and p1 are as defined above,
- R²¹ represents, independently at each occurrence p1, a hydrogen atom, a C₁-C₅ alkyl group, a group -T⁵-NH-E^{∗} in which T⁵ represents a C₂-C₅ alkylene group, or a single bond*,
- E is a 3-amino-, or 4-amino-3-cyclobutene-1,2-dione group, or a C₁-C₅ alkyl group.

In formula (VII), ^{∗} indicates the points of attachment of said linker L with groups R.

### R²¹ group

The C₁-C₅ alkyl group as R²¹ group can be a linear alkyl group or a branched alkyl group, and preferably a linear alkyl group.

The C₁-C₅ alkyl group is preferably a C₁-C₃ alkyl group, and more preferably a methyl or ethyl group.

The C₂-C₅ alkylene group as T⁵ can be a linear or branched alkylene group, and preferably a linear alkylene group.

The C₂-C₅ alkylene group as T⁵ is preferably a C₂-C₄ alkylene group.

When R²¹ is a single bond, the nitrogen to which R²¹ is attached is directly linked to a group R.

In one preferred embodiment, R²¹ represents, independently at each occurrence p1, a hydrogen atom or a group -T⁵-NH-E^{∗}.

In the linker L of formula (VII), there may be alternation of a radical - [NR²¹-(CR¹⁸R¹⁹)_{q1}] where R²¹ represents a hydrogen atom, and of a radical - [NR²¹-(CR¹⁸R¹⁹)_{q1}] where R²¹ represents a group -T⁵-NH-E^{∗}.

### E group

The C₁-C₅ alkyl group as E group can be a linear alkyl group or a branched alkyl group, and preferably a linear alkyl group.

The C₁-C₅ alkyl group is preferably a C₁-C₃ alkyl group, and more preferably a methyl or ethyl group.

In one particular preferred embodiment, the linker L responds to any one of the following formula (VII): 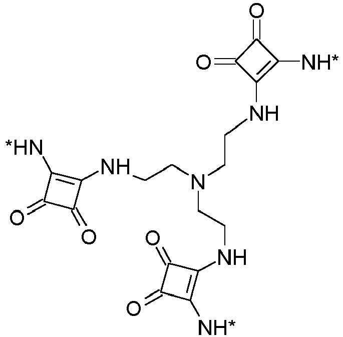 wherein * represent the possible points of attachment of the linker L to the group R.

### The linker L can be a group responding to the following formula (VIII):

^{∗}(CR²²R²³)ₘ₂-NR²⁸-[(CR²⁴R²⁵)_{q2}-NR²⁹]ₚ₂-(CR²⁶R²⁷)ₘ₃^{∗} (VIII)

in which:
- R²² and R²³ represent, independently from each other, independently at each occurrence m2, a hydrogen atom or a C₁-C₅ alkyl group,
- m2 is an integer ranging from 1 to 5,
- R²⁴ and R²⁵ represent, independently from each other, independently at each occurrence q2, independently at each occurrence p2, a hydrogen atom or a C₁-C₅ alkyl group,
- R²⁶ and R²⁷ represent, independently from each other, independently at each occurrence m3, a hydrogen atom or a C₁-C₅ alkyl group,
- m3 is an integer ranging from 1 to 5,
- R²⁸ represents a hydrogen atom, a C₁-C₅ alkyl group, a C₁-C₅ alkylene group^{∗}, or a single bond^{∗},
- R²⁹ represents, independently at each occurrence p2, a hydrogen atom, a C₁-C₅ alkyl group, a C₁-C₅ alkylene group^{∗}, a single bond^{∗}, or a - [(CR²⁴R²⁵)_{q2}-NR²⁹]ₚ₂-(CR²⁶R²⁷)ₘ₃^{∗} group,
- q2 represents, independently at each occurrence p2, an integer ranging from 2 to 5, and
- p2 is an integer ranging from 0 to 4.

In formulae (VIII), * indicates the possible points of attachment of said linker L with groups R.

### R²², R²³, R²⁴, R²⁵, R²⁶, R²⁷, R²⁸ and R²⁹ groups

The C₁-C₅ alkyl group as R²² group (respectively as R²³, R²⁴, R²⁵, R²⁶, R²⁷, R²⁸ or R²⁹ group) can be a linear alkyl group or a branched alkyl group, and preferably a linear alkyl group.

The C₁-C₅ alkyl group is preferably a C₁-C₃ alkyl group, and more preferably a methyl or ethyl group.

When R²⁸ (respectively R²⁹) is a single bond, the nitrogen to which R²⁸ (respectively R²⁹) is attached is directly linked to a group R.

In one preferred embodiment, R²⁸ represents a hydrogen atom or a C₁-C₅ alkylene group^{∗}.

In one preferred embodiment, R²⁹ represents, independently at each occurrence p2, a hydrogen atom or a C₁-C₅ alkylene group^{∗}.

At least one of R²² and R²³ groups is preferably a hydrogen atom, both R²² and R²³ groups are more preferably hydrogen atoms.

For all occurrences m2, the groups CR²²R²³ are preferably identical.

At least one of R²⁴ and R²⁵ groups is preferably a hydrogen atom, both R²⁴ and R²⁵ groups are more preferably hydrogen atoms.

For all occurrences q2, the groups CR²⁴R²⁵ are preferably identical.

At least one of R²⁶ and R²⁷ groups is preferably a hydrogen atom, both R²⁶ and R²⁷ groups are more preferably hydrogen atoms.

For all occurrences m3, the groups CR²⁶R²⁷ are preferably identical.

In the linker L of formula (VIII), the radical [(CR²⁴R²⁵)_{q2}-NR²⁹] may be identical for all occurrences p2, preferably with R²⁹ representing a C₁-C₅ alkylene group*.

In one particularly preferred embodiment, the linker L responds to any one the following formula (VIII): 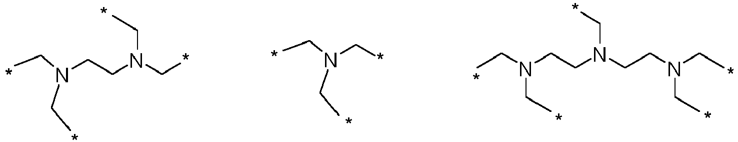 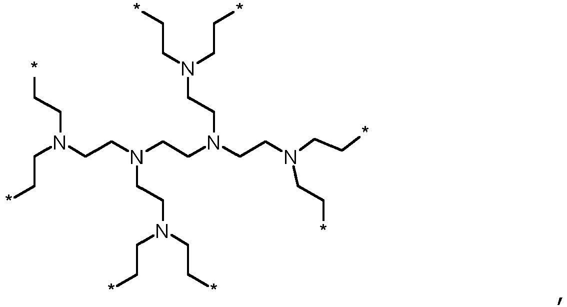 wherein * indicates the possible points of attachment of said linker L with groups R.

### The linker L can be a group responding to the following formula (IX):

^{∗}(CR³⁰R³¹)ₘ₄-NR³⁴-[(CH₂-CH₂-O)_{q4}-CH₂-CH₂-NR³⁵]ₚ₄-(CR³²R³³)ₘ₅^{∗} (IX)

in which:
- R³⁰ and R³¹ represent, independently from each other, independently at each occurrence m4, a hydrogen atom or a C₁-C₅ alkyl group,
- m4 is an integer ranging from 1 to 5,
- R³² and R³³ represent, independently from each other, independently at each occurrence m5, a hydrogen atom or a C₁-C₅ alkyl group,
- m5 is an integer ranging from 1 to 5,
- R³⁴ represents a hydrogen atom, a C₁-C₅ alkyl group, a C₁-C₅ alkylene group^{∗}, or a single bond^{∗},
- R³⁵ represents, independently at each occurrence p4, a hydrogen atom, a C₁-C₅ alkyl group, a C₁-C₅ alkylene group^{∗}, or a single bond^{∗},
- q4 represents, independently at each occurrence p4, an integer ranging from 1 to 5, and
- p4 is an integer ranging from 0 to 4.

In formula (IX), * indicates the points of attachment of said linker L with groups R.

### R³⁰, R³¹, R³², R³³, R³⁴, and R³⁵ groups

The C₁-C₅ alkyl group as R³⁰ group (respectively as R³¹, R³², R³³, R³⁴, or R³⁵ group) can be a linear alkyl group or a branched alkyl group, and preferably a linear alkyl group.

The C₁-C₅ alkyl group is preferably a C₁-C₃ alkyl group, and more preferably a methyl or ethyl group.

When R³⁴ (respectively R³⁵) is a single bond, the nitrogen to which R³⁴ (respectively R³⁵) is attached is directly linked to a group R.

In one preferred embodiment, R³⁴ represents a hydrogen atom or a C₁-C₅ alkylene group^{∗}.

In one preferred embodiment, R³⁵ represents, independently at each occurrence p4, a hydrogen atom or a C₁-C₅ alkylene group^{∗}.

At least one of R³⁰ and R³¹ groups is preferably a hydrogen atom, both R³⁰ and R³¹ groups are more preferably hydrogen atoms.

For all occurrences m4, the groups CR³⁰R³¹ are preferably identical.

At least one of R³² and R³³ groups is preferably a hydrogen atom, both R³² and R³³ groups are more preferably hydrogen atoms.

For all occurrences m5, the groups CR³²R³³ are preferably identical.

In one particularly preferred embodiment, the linker L responds to the following formula (IX): 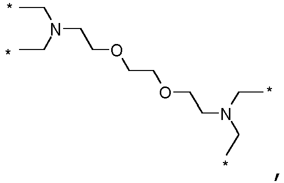 wherein * indicates the possible points of attachment of said linker L with groups.

The linker L can be a polyazacycloalkane saturated group in which at least two -N- functions represent points of attachment of said linker L with groups R. The polyazacycloalkane saturated group can comprise "free" -N-function(s), i.e. not linked to said groups R so that they are in the form of -NH functions, which the proviso that at least two amino functions are linked/bounded to R groups.

The polyazacycloalkane saturated group as linker L can be selected from cyclen, cyclam, and derivatives thereof.

The polyazacycloalkane saturated group can comprise from 3 to 25 carbon atoms, and preferably from 4 to 15 carbon atoms.

The polyazacycloalkane saturated group as linker L has preferably at least three, and more preferably at least for -N- functions represent points of attachment of said linker L with groups R.

In particular, the polyazacycloalkane saturated group as linker L can respond to any one of the following formulae: 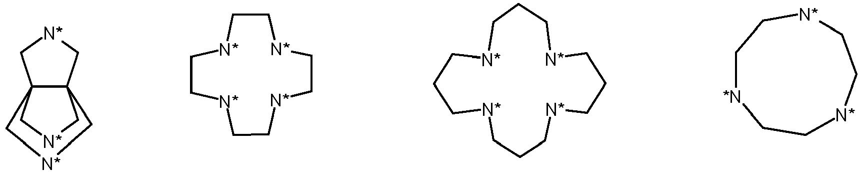 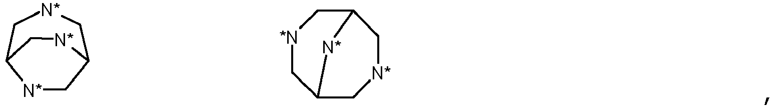 wherein * indicates the possible points of attachment of said linker L with groups.

As an example, said latter linker can have one -N- function in excess of two which is not attached to a group R according to the following formula: 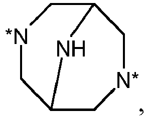 wherein * indicates the points of attachment of said linker L with groups.

The linker L can be a polyaminoacid group in which at least two functions represent points of attachment of said linker L with groups R, said function being a carbonyl function (derived from the carboxylic acid function), a thiol function, or an amine function.

These functions are "free" functions or pending functions within the polyaminoacid (i.e. not participating to the polymerization involving reaction of an amino function with a carboxylic acid function so as to form said polyaminoacid).

The polyaminoacid group may comprise several aminoacid selected from lysine, ornithine, arginine, histidine, aspartic acid, glutamic acid, tryptophan, and cysteine.

Examples of such polyaminoacid groups are the ones based on polyglutamic acid (PGA) or aspartic acid, such as the following one based on aspartic acid: 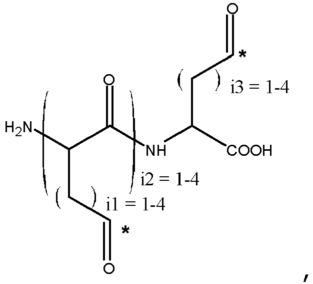 wherein * indicates the possible points of attachment of said linker L with groups.

### The linker L can be a polyamidoamine group.

A poly(amidoamine) group is also called a PAMAM group. It is a class of dendrimer which is made of repetitively branched subunits of amide and amine functionality.

In one preferred embodiment, the poly(amidoamine) group responds to the following formula: 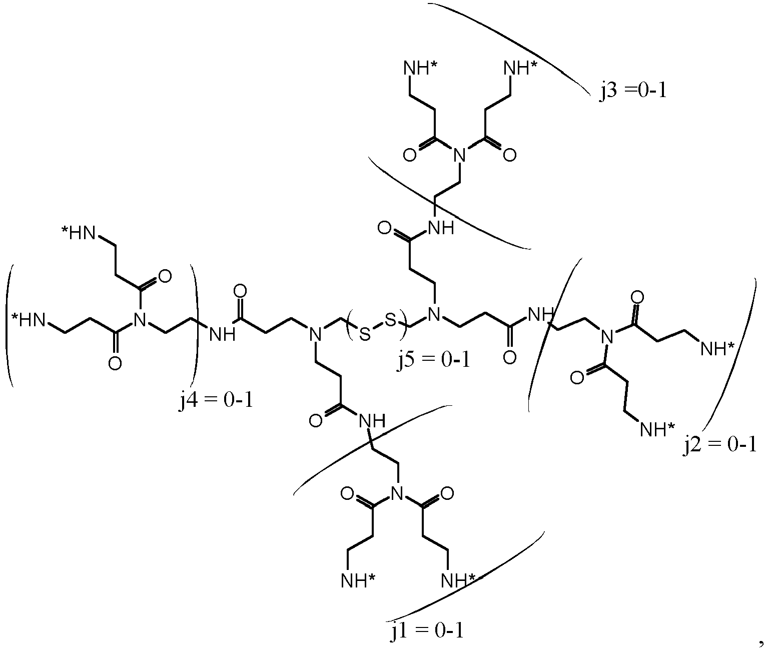 wherein * indicates the possible points of attachment of said linker L with groups.

In one particularly preferred embodiment of the present invention, the ligand is selected from the following formulae (I-a) to (I-i):

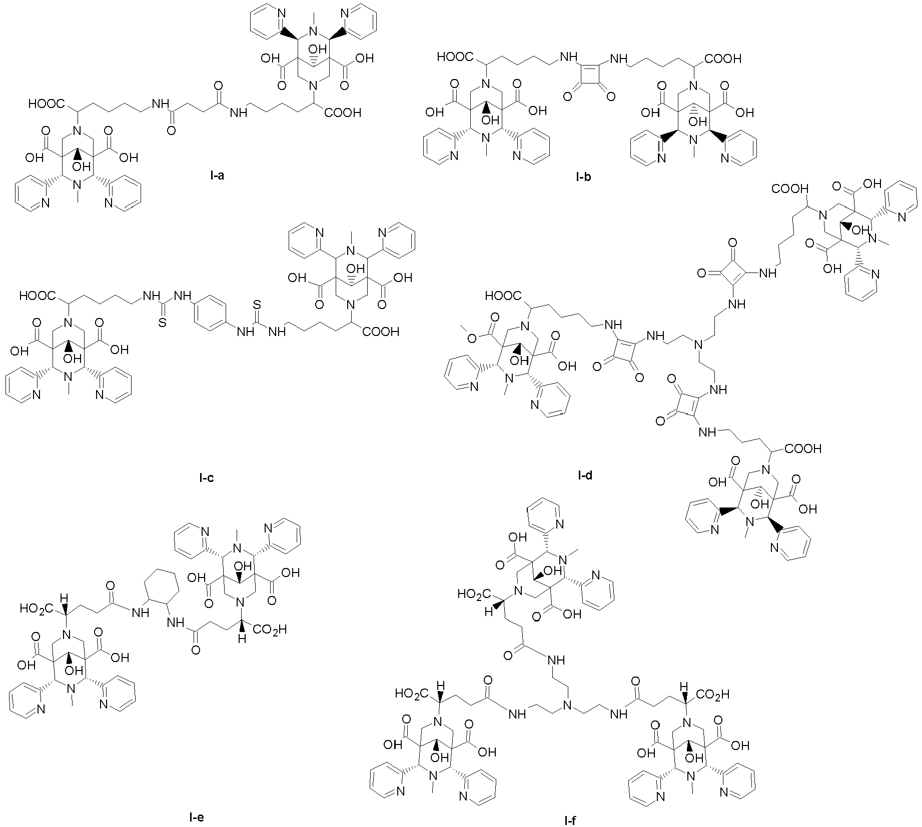

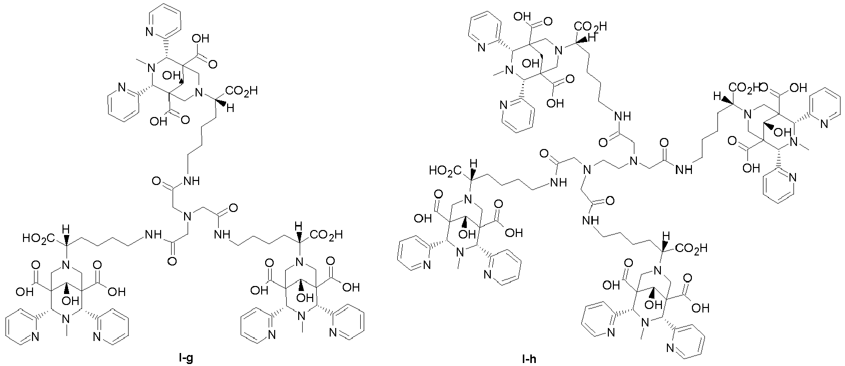

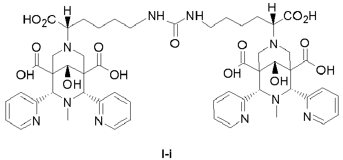

A second object of the present invention is a complex of a metal M, wherein said complex comprises several metal ions of said metal M complexed with a bispidine-based metal chelating ligand (I) as defined in the first object of the present invention, said metal M being selected from copper, manganese, gallium, cobalt, zinc, nickel, and iron, and preferably selected from copper and manganese.

The metal M can be radioactive.

The metal ion can be selected from Mn²⁺, Mn³⁺, Cu²⁺, Cu⁺, Ga³⁺, Co²⁺, Co³⁺, Fe²⁺, Fe³⁺, Zn²⁺, or Ni²⁺.

The complex of the present invention exhibits high relaxivity, show a favorable pharmacokinetic profile, is completely excreted, is chemically stable, exhibit high water solubility, offer the possibility of providing safe alternatives to traditional GBCAs and offer the potential for a significant dose reduction in comparison to state-of-the-art Mn-based contrast agents (CAs), and is suitable for imaging of different body regions.

A third object of the present invention is the use of a bispidine-based metal chelating ligand (I) as defined in the first object of the present invention or a complex as defined in the second object of the present invention, in the field of medical imaging or therapy, and more specifically as MRI (magnetic resonance imaging) contrast agents and/or nuclear imaging agents for PET (positron emission tomography) or SPECT (single photon emission tomography).

Positron emission tomography (PET) is a highly sensitive imaging technique with high tissue penetration. This technique can allow for the *in vivo* imaging of diseased tissues by targeting biochemical processes; thus allowing for detection of disease before physical changes occur.

More particularly, the bispidine-based metal chelating ligand (I) as defined in the first object of the present invention can be used as a chelator for radioactive copper for application in immuno-positron emission tomography (PET).

More particularly, the bispidine-based metal chelating ligand (I) as defined in the first object of the present invention can be used as bifunctional chelating agents for HER2+ immuno-imaging of breast cancer or for PET immuno-imaging of multiple myeloma, for radioimmunotherapy or again as a theranostic probe combining therapy and diagnostic.

The present invention is illustrated in more detail in the examples below, but it is not limited to said examples.

### Examples

Solvents and starting materials were purchased from Aldrich, Acros and Alfa Aesar and used without further purification. IR spectra were recorded on a Perkin Elmer Spectrum One Spectrophotometer as solid samples and only the most significant absorption bands are given in cm⁻¹. Elemental analyses and mass spectrometry analysis were carried out by the Service Commun d'Analyses of the University of Strasbourg. ¹H and ¹³C NMR spectra and 2D COSY, NOESY, HSQC, and HMBC experiments were recorded on Avance 300 and Avance 400 spectrometers operating at 7.04 T and 9.39 T, respectively. Chemical shifts are reported in ppm, with residual protonated solvent as internal reference.

### Example 1: synthesis of intermediate compound 4

Compound **4** was prepared according to the following scheme 2:

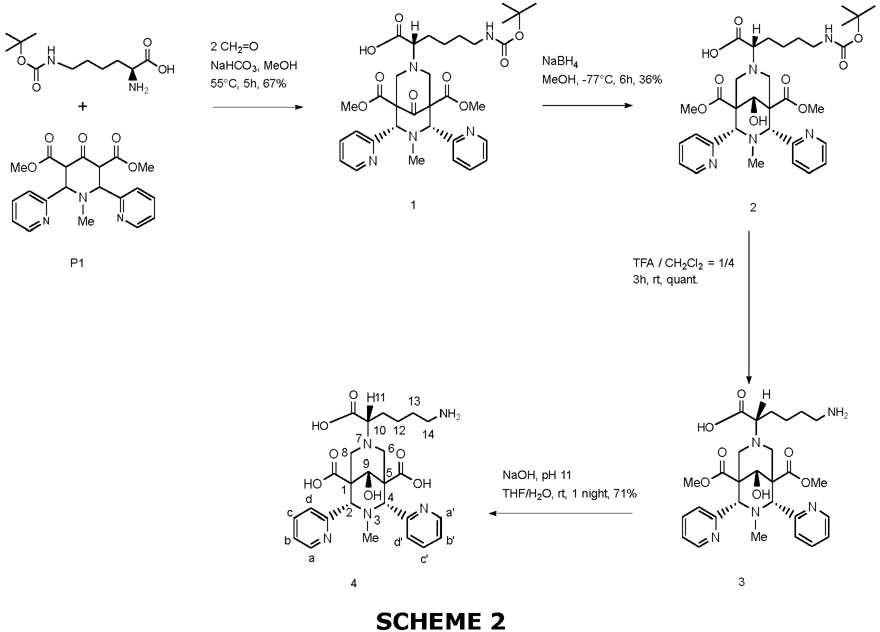

**P1** is a well-known starting material to lead to bispidone and bispidine compounds.

*Bispidone **1.*** In a solution of methanol (60 mL), Nε-Boc-L-lysine (4.19 g, 17.40 mmol) and NaHCO₃ (1.46 g, 17.40 mmol) were mixed under stirring during 1h at 45°C. Then, formaldehyde (3.5 mL, 47.50 mmol) and precursor **P1** (6.06 g, 15.80 mmol) in methanol (20 mL) were added at room temperature (rt) and the mixture was stirred under reflux during 5h. At the end of the reaction, the solution was filtered and solvents were removed under reduced pressure. The obtained solid was dissolved in a minimum of methanol and was precipitated upon dropwise addition in a large amount of diethyl ether. After centrifugation, precipitates were collected and dried to give a pure beige solid (6.93 g, 67%).
TLC (Al₂O₃; DCM/MeOH, 85/15); R*f* = 0.5.
¹H-NMR (400 MHz, CDCl₃): δ 8.95 (m, 2H, Ha + Ha'), 7.70 (t, *J*= 7.6 Hz, 1H, Hc'), 7.59 (t, *J*= 7.5 Hz, 1H, Hc), 7.35 (m, 1H, Hb'), 7.17 (m, 3H, Hd'+Hd+Hb), 4.67 (s, 1H, H2), 4.56 (s, 1H, H4), 3.81 (broad s, 1H, COOH), 3.77 (s, 3H, OCH₃), 3.66 (s, 3H, OCH₃), 3.22 (AB system, δ_{A} = 3.60, δ_{B} = 2.84, *J_{AB}* = 12.0 Hz, v_{A} = 1440 Hz, v_{B} = 1136 Hz, 2H, H8/H6), 2.96 (AB system, δ_{A} = 3.29, δ_{B} = 2.62, *J_{AB}* = 12.0 Hz, v_{A} = 1316 Hz, v_{B} = 1048 Hz, 2H, H8/H6), 2.99 (m, 2H, H14), 2.57 (d, 1H, H10), 1.86 (s, 3H, CH₃), 1.64-1.22 (m, 15H, H11/H12/H13/OC(CH₃)₃).
¹³C-NMR (100 MHz, CDCl₃): δ 202.7 (C9), 177.0 (COOH), 168.0 (COOMe), 167.5 (COOMe), 156.1 (3C, 1Cpy+Cpy'+COO*t*Bu), 151.5 (Ca/Ca'), 151.1 (Ca/Ca'), 137.5 (Cc/Cc'), 137.0 (Cc/Cc'), 124.5 (Cb/Cb'/Cd/Cd'), 124.2 (Cb/Cb'/Cd/Cd'), 124.0 (Cb/Cb'/Cd/Cd'), 121.7 (Cb/Cb'/Cd/Cd'), 78.7 (C(OCH₃)₃), 74.0 (C10), 72.6 (2C, C2+C4), 63.0 (2C, C1+C5), 57.5 (C6/C8), 54.7 (C6/C8), 52.8 (OCH₃), 52.5 (OCH₃), 43.0 (CH₃), 40.3 (C14), 29.9 (C11), 28.9 (C13), 28.5 (C(*C*H₃)₃), 24.0 (C12).
IR (cm⁻¹, ATR) ν 3366 (broad, N-H amide), 2943, 2860 (broad, O-H acid), 1739 (s, C=O ester), 1695 (s, C=O acid), 1572 (s, C=C aromatic), 1254 (s, C-O).

Electrospray ionization ESI/MS⁺: *m*/*z* = 654.31 ([M+H]⁺, 100%), 655.31 ([M+H]⁺, 35.7%), 656.32 ([M+H]⁺, 6.2%), 657.32 ([M+H]⁺, 0.7%).

*Bispidol **2**.* Bispidone **1** (3.08 g, 4.71 mmol) was dissolved in anhydrous methanol (110 mL) and was cooled at -77°C on a bath of acetone/dry ice. Then, sodium borohydride (240.00 mg, 7.07 mmol) was slowly added. After 6h at - 77°C, the mixture was placed at 4°C for one night. Then NH₄Cl (520.00 mg, 9.72 mmol) was added in the flask and the mixture was stirred during 10 minutes. Solvents were removed under reduced pressure and the obtained solid was purified by flash chromatography column (reverse phase, CH₃CN/H₂O 20/80-25/75). The pure compound **2**.2H₂O was obtained as a white solid (1.10 g, 32%).
TLC (SiO₂; DCM/MeOH, 80/20); R*f* = 0.3-0.5.
¹H-NMR (400 MHz, MeOD): δ= 8.67 (m, 2H, Ha+Ha'), 7.81 (m, 2H, Hc+Hc'), 7.60 (m, 2H, Hd+Hd'), 7.38 (m, 2H, Hb+Hb'), 4.89 (s, 2H, H2+H4), 4.50 (s, 1H, H9), 4.30 (AB system, δ_{A} = 4.60, δ_{B} = 4.00, *J_{AB}* = 12.0 Hz, 2H, H6/H8), 3.96 (AB system, δ_{A} = 4.10, δ_{B} = 3.91, *J_{AB}* = 12.0 Hz, 2H, H6/H8), 3.76 (m, 1H, H10), 3.63 (s, 3H, OCH₃), 3.55 (s, 3H, OCH₃), 3.08 (m, 2H, H14), 2.33-2.17 (m, 2H, H11), 1.73 (m, 4H, H12+H13), 1.65 (s, 3H, CH₃), 1.39 (s, 9H, C(*C*H₃)₃).
¹³C-NMR (75 MHz, MeOD): δ 172.8 (COOH), 170.4 (2C, COOMe), 158.3 (COO*t*Bu/Cpy/Cpy'), 157.8 (COO*t*Bu/Cpy/Cpy'), 157.7 (COO*t*Bu/Cpy/Cpy'), 150.6 (Ca/Ca'), 150.1 (Ca/Ca'), 138.1 (Cc/Cc'), 138.0 (Cc/Cc'), 128.6 (2C, Cd+Cd'), 124.9 (Cb/Cb'), 124.8 (Cb/Cb'), 79.7 (*C*(CH₃)₃), 73.8 (C9), 72.0 (C10), 67.8 (C2/C4), 67.5 (C2/C4), 56.8 (C8/C6), 53.0 (OCH₃), 52.9 (OCH₃), 52.3 (2C, C1+C5), 51.2 (8/6), 41.6 (CH₃), 41.1 (C14), 30.7 (C13), 30.0 (C11), 28.7 (C(*C*H₃)₃), 25.1 (C12).

Electrospray ionization ESI/MS⁺: *m*/*z* = 656.33 ([M+H]⁺, 100%), 657.34 ([M+H]⁺, 35.7%), 658.34 ([M+H]⁺, 3.5%).

Elemental analysis calculated for C₃₃H₄₅O₉N₅.1.5H₂O: C, 58.05, H, 7.09, N, 10.26. Found: C, 57.82, H, 6.81, N, 10.38.

*Bispidol **3.*** Bispidol **2** (413.0 mg, 0.74 mmol) was dissolved in CH₂Cl₂ (8 mL) and trifluoroacetic acid (2 mL) and the mixture was stirred at room temperature. After 3h, solvents were evaporated under reduced pressure and the crude TFA salt was used in the next step without purification (413 mg).
TLC (C₁₈; CH₃CN/H₂O, 0.1% TFA, 50/50); R*f* = 0.8.
¹H-NMR (400 MHz, MeOD): δ 8.76 (m, 2H, Ha+Ha'), 7.94 (m, 2H, Hc+Hc'), 7.77 (m, 2H, Hd+Hd'), 7.52 (m, 2H, Hb+Hb'), 5.63 (s, 1H, H2/H4), 5.61 (s, 1H, H2/H4), 4.41 (s, 1H, H9), 3.72 (s, 3H, OCH₃), 3.69 (s, 3H, OCH₃), 3.33 (AB system, δ_{A} = 3.52, δ_{B} = 3.13, *J_{AB} =* 12.0 Hz, v_{A} = 1408 Hz, v_{B} = 1252 Hz, 2H, H6/H8), 3.39 (AB system, δ_{A} = 3.41, δ_{B} = 3.38, *J_{AB} =* 10.5 Hz, v_{A} = 1363 Hz, v_{B} = 1353 Hz, 1H, H10), 3.13 (m, 2H, H6/H8), 3.03 (t, *J*= 8.5Hz, 2H, H14), 2.35-1.92 (m, 2H, H11), 2.31 (s, 3H, CH₃), 1.90 (m, 2H, H13), 1.61 (m, 2H, H12).
¹³CNMR (75 MHz, D₂O): δ 173.6 (COOH), 170.5 (COOMe), 170.4 (COOMe), 152.9 (Cpy/Cpy'), 152.8 (Cpy/Cpy'), 151.0 (Ca/Ca'), 150.4 (Ca/Ca'), 139.1 (Cc/Cc'), 139.0 (Cc/Cc'), 129.0 (Cd/Cd'), 128.6 (Cd/Cd'), 126.2 (Cb/Cb'), 126.1 (Cb/Cb'), 73.9 (C9), 68.1 (C10), 67.6 (C2/C4), 67.1 (C2/C4), 55.8 (C6/C8), 54.4 (C1/C5), 53.7 (C1/C5), 53.3 (OCH₃), 53.2 (OCH₃), 50.4 (C6/C8), 43.4 (CH₃), 40.7 (C14), 29.8 (C11), 28.8 (C13), 25.0 (C12).

*Compound **4.*** Bispidol **3** (279.0 mg, 0.50 mmol) was dissolved in a mixture of H₂O/THF (8 mL/2 mL) and sodium hydroxide (240.0 mg, 3.0 mmol) was added at room temperature under stirring. After 48h, the mixture was evaporated under reduced pressure and was purified by column chromatography (reverse phase, 100% H₂O to 100% MeOH in 15 min).

Pure compound 4.2Na (265.0 mg, 71%) was obtained as a white powder.
TLC (C₁₈; CH₃CN/H₂O, 20/80), R*f* = 0.4.
¹H-NMR (400 MHz, D₂O): δ 8.48 (m, 2H, Ha+Ha'), 7.54 (m, 2H, Hc+Hc'), 7.30 (d, *J*= 9.0 Hz, 1H, Hd/Hd'), 7.19 (m, 1H, Hd/Hd'), 7.14 (m, 2H, Hb+Hb'), 4.40 (s, 1H, H2/H4), 4.31 (s, 1H, H2/H4), 3.82 (s, 1H, H9), 2.42 (AB system, δ_{A} = 2.92, δ_{B} = 1.93, *J_{AB}* = 12.3 Hz, v_{A} = 1167 Hz, v_{B} = 774 Hz, 2H, H6/H8), 2.36 (t, *J*= 6.9 Hz, 2H, H14), 2.29 (AB system, δ_{A} = 2.75, δ_{B} = 1.83, *J_{AB}* = 11.2 Hz, v_{A} = 1102 Hz, v_{B} = 731 Hz, 2H, H6/H8), 2.20 (m, 1H, H10), 1.51 (s, 3H, CH3), 1.47-1.36 (m, 2H, H11), 1.20 (m, 2H, H13), 1.10 (m, 2H, H12).
¹³C-NMR (100 MHz, D₂O): δ 181.4 (COOH), 179.1 (COOH), 178.6 (COOH), 159.9 (Cpy/Cpy'), 159.3 (Cpy/Cpy'), 149.2 (2C, Ca+Ca'), 137.0 (2C, Cc+Cc'), 126.0 (Cd/Cd'), 125.5 (Cd/Cd'), 122.9 (Cb/Cb'), 122.8 (Cb/Cb'), 75.3 (C10), 74.3 (C9), 67.8 (C2/C4), 67.1 (C2/C4), 55.9 (C6/C8), 53.3 (C5/C8), 51.7 (C1/C5), 51.5 (C1/C5), 42.8 (CH3), 40.2 (C14), 32.0 (C13), 29.6 (C11), 23.0 (C12).
IR (cm⁻¹, ATR) ν 3342 (broad, O-H alcool/N-H amine), 2949 (broad, O-H acid), 1584, 1568 (s, C=C aromatic/ N-H amine), 1366 (s, C-O).

Electrospray ionization ESI/MS⁺: *m*/*z* = 528.25 ([M+H]⁺, 100%), 529.25 ([M+H]⁺, 28.1%), 530.25 ([M+H]⁺, 3.8%).

Elemental analysis calculated for C₂₆H₃₁O₇N₅Na2.3H₂O: C, 49.91, H, 5.96, N, 11.19. Found: C, 49.79, H, 5.92, N, 11.19.

### Example 2: synthesis of compound (I-a)

Compound **(I-a)** was prepared according to the following scheme 2:

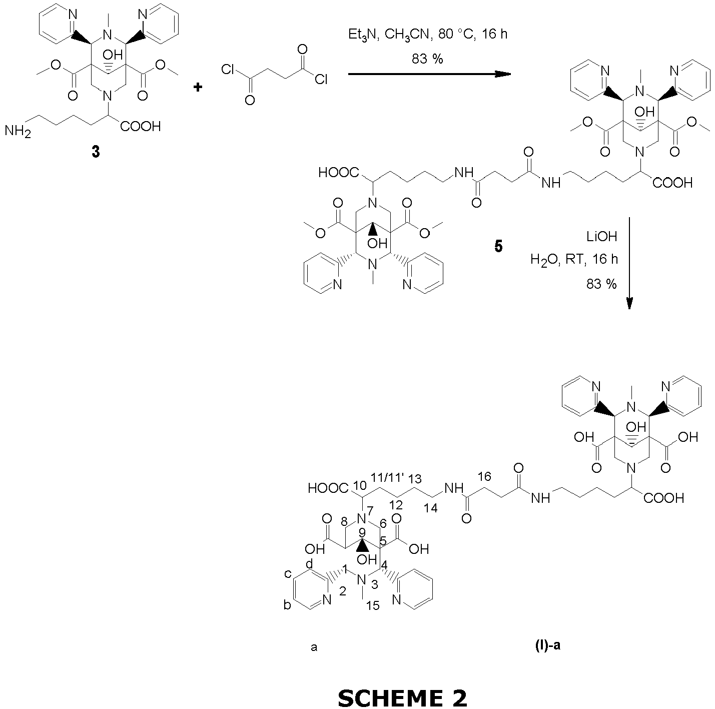

*Compound **5.*** Compound **3** (191.3 mg, 0.25 mmol) is dissolved in 15 ml of CH₃CN. Triethylamine (0.153 ml, 1.1 mmol) is then added and the mixture is placed under argon. Succinyl chloride (14.4 mmol, 0.088 mmol) in 5 ml of CH₃CN is added dropwise to the mixture under argon and the middle is stirred at 80 °C for 16 hours. At the end the solvent is evaporated and the crude product is purified on SPOT II FPLC (C₁₈, CH₃CN/H₂O, 0.1 % TFA) to give 5 (104 mg) with 83 % yields.
¹H NMR (MeOD, 400 MHz) : *δ* 8.77 (m, 4H, Ha+Ha'), 7.93 (m, 4H Hc + Hc'), 7.75 (m, 4H, Hd + Hd'), 7.52 (m, 4H, Hb + Hb'), 5.62 (s, 2H, H2/H4), 5.60 (s, 2H, H2/H4), 4.40 (s, 2H, H9), 3.71 (s, 6H, OCH₃), 3.68 (s, 6H, OCH₃), 3.54 (d, J = 12.4 Hz, 2H, H6/H8), 3.37 (dd, J = 10.1, 4.4 Hz, 2H, H10) , 3.26 (m, 4H, H14), 3.12 (m, 6H, H6/H8), 2.51 (s, 4H, H16), 2.29 (m, 8H, H15 + H11/H11'), 1.86 (m, 2H, H11/11'), 1.71 (m, 4H, H13), 1.54 (m, 4H, H12).
**(I-a).** Compound **5** (104 mg, 0.073 mmol) and LiOH (33 mg, 1.38 mmol are dissolved in 15 ml of H₂O. The mixture was stirred for 16 hours at room temperature. At the end the solvent is evaporated. The crude product was dissolved in a minimum of water and the pH was adjusted to 2 and the solution was purified on SPOT II FPLC (C₁₈, CH₃CN/H₂O, 0.1 % TFA) to give **(I)-a** with 83 % yield.
¹H NMR (MeOD, 400 MHz): *δ* 8.77 (m, 4H, Ha+Ha'), 7.91 (m, 4H Hc + Hc'), 7.76 (m, 4H, Hd + Hd'), 7.50 (m, 4H, Hb + Hb'), 5.57 (s, 2H, H2/H4), 5.55 (s, 2H, H2/H4), 4.40 (s, 2H, H9), 3.46 (m, 2H, H6/H8), 3.35 (m, 2H, H10), 3.27 (m, 4H, H14), 3.07 (m, 6H, H6/H8), 2.51 (s, 4H, H16), 2.30 (m, 8H, H15 + H11/H11'), 1.84 (m, 2H, H11/11'), 1.70 (m, 4H, H13), 1.54 (m, 4H, H12).
¹³C NMR (MeOD, 126 MHz): *δ* 174.68, 173.82, 171.78, 171.63, 153.24, 153.16, 150.85, 150.33, 138.91, 138.79, 129.12, 128.76, 126.01, 74.07, 68.40, 67.84, 67.32, 56.28, 53.31, 52.79, 50.44, 43.41, 40.42, 32.41, 30.53, 29.94, 25.38.
ESI/MS⁻: *m*/*z* = 1135.48 ([M - H]⁻, 100 %), 1136.48 ([M - H]⁻, 75 %), 1137.48 ([M - H]⁻, 29 %), ([M - H]⁻, 8 %), 567.23 ([M - 2H]2⁻, 100 %), 567.73 [M - 2H]2⁻, 68 %), 568.23 ([M - 2H]2⁻, 26 %), 568.73 ([M - 2H]2⁻, 5 %)

### Example 3: synthesis of compound (I-b)

Compound **(I-b)** was prepared according to the following scheme 3:

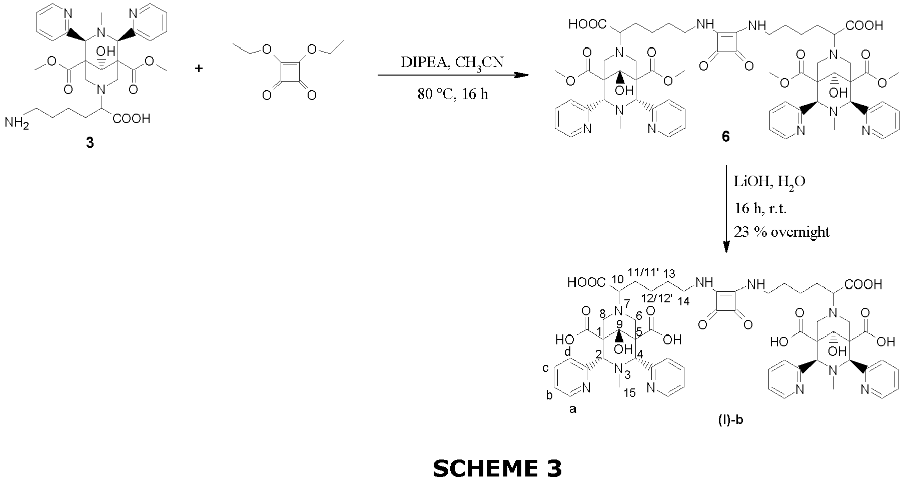

*Compound* **6.** Compound **3** (72.4 mg, 0.061 mmol) and DIPEA (0.083 µl, 0.48 mmol) are dissolved in ACN (10 ml) and the middle is placed under argon. Diethyl squarate (16.1 mg 0.095 mmol) dissolved in a minimum of ACN is then added end the reaction is heated at 50 °C for 2 hours. Compound **3** (113 mg, 0.15 mmol) and DIPEA (0.12 µl, 0.69 mmol) a second time and the temperature is increased to 80 °C and heated overnight. At the end of the reaction the solvent is removed under vacuum. The crude product is finally purified on C₁₈ (H₂O, ACN, 0.1 % TFA) to give compound **6** (15 mg).
¹H NMR (500 MHz, MeOD) δ 8.75 (m, 4H, Ha + Ha'), 7.91 (m, 4H, Hc + Hc'), 7.72 (m, 4H, Hd + Hd'), 7.49 (m, 4H, Hb + Hb'), 5.58 (s, 2H, H2 + H4) 5.57 (s, 2H, H2 + H4), 4.39 (s, 2H, H9), 3.67 (m, 14H, OCH₃ + H14), 3.61 (m, 2H, H6 + H8), 3.40 (dd, *J₁* = 9.1, *J₂* = 5.3 Hz, 2H, H10), 3.14 (m, 6H, H6 + H8), 2.33 (m, 2H, H11/11'), 2.27 (s, 6H, H15), 1.86 (m, 6H, H11/11' + H13) 1.69 (m, 2H, H12/H12'), 1.61 (m, 2H, H12/H12').
¹³C NMR (126 MHz, MeOD) δ 173.69, 170.45, 170.36, 169.44, 153.06, 152.91, 150.96, 150.51, 138.93, 138.83, 129.03, 128.70, 126.14, 126.09, 74.01, 68.37, 67.76, 67.20, 56.16, 53.69, 53.28, 53.21, 50.29, 45.19, 43.19, 32.50, 29.82, 25.19.
MALDI-MS: *m*/*z* = calculated for C₆₀H₇₂N₁₀O₁₆ [M+] 1188.51, found 1188.14.

*Compound **(I)-b**.* Compound **6** (15 mg, 0.013 mmol) and LiOH (6 mg, 0.25 mmol) were dissolved in 3 ml of water. The mixture was stirred at room temperature for 3 days. At the end, the solvent was removed under vacuum. The crude product was dissolved in a minimum of water and the pH was adjusted to 2 and purified on C₁₈ (H₂O/ ACN, 0.1 % TFA) to give **(I)-b** (9 mg) with 64 % yields.
¹H NMR (400 MHz, MeOD) δ 8.74 (td, *J₁* = 7.8, *J₂* = 1.8 Hz, 4H, Ha + Ha'), 7.90 (m, 4H, Hc + Hc'), 7.76 (m, 4H, Hd + Hd'), 7.49 (m, 4H, Hb + Hb'), 5.56 (s, 2H, H2 + H4), 5.54 (s, 2H, H2 + H4), 4.42 (s, 2H, H9), 3.73 (m, 4H, H14), 3.54 (dd, *J₁* = 12.4, *J₂* = 2.1 Hz, 2H, H6 + H8), 3.38 (dd, *J₁* = 9.3, *J₂* = 5.0 Hz, 2H, H10), 3.10 (m, 6H, H6 + H8), 2.33 (m, 8H, H15 + H11/11') 1.86 (m, 6H, H11/11' + H13), 1.65 (m, 4H, H12/H12').
¹³C NMR (101 MHz, MeOD) δ 172.29, 169.04, 168.95, 168.04, 151.66, 151.50, 149.56, 149.11, 137.53, 137.42, 124.73, 124.69, 116.75, 114.46, 72.61, 66.97, 66.35, 65.79, 54.75, 52.22, 51.94, 51.36, 48.89, 43.84, 41.91, 31.15, 28.50, 23.77.
MALDI-MS: *m*/*z* = calculated for C₅₆H₆₄N₁₀O₁₆ [M⁺] 1132.45, found 1132.14.

### Example 4: synthesis of compound (I-c)

Compound **(I-c)** was prepared according to the following scheme 4:

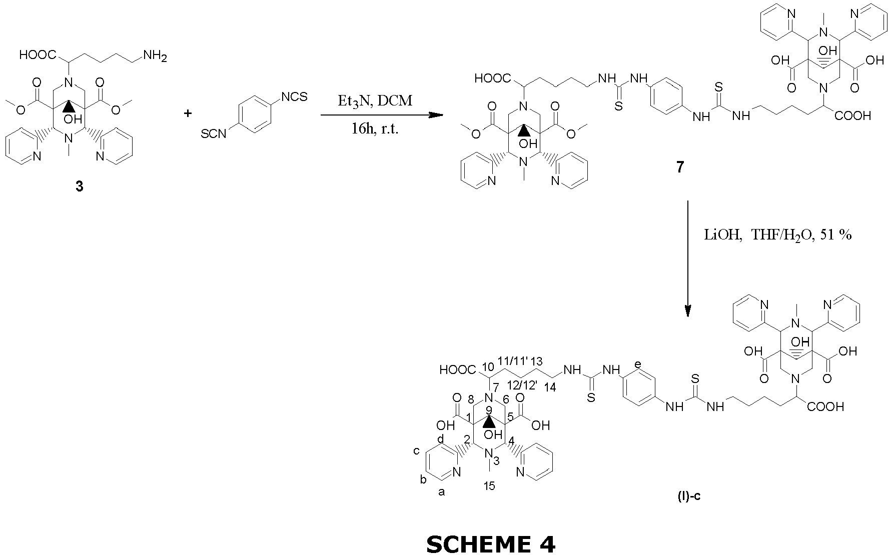

*Compound **7.*** Compound **3** (58 mg, 8.68×10⁻⁵mol, 2.3 eq.) was suspended in 2 mL of DCM. P-phenyl diisothiocyanate (7 mg, 3.70×10⁻⁵ mol, 1 eq.) and triethylamine (17.5 µL, 3.62×10⁻⁴ mol, 10 eq.) were added to the solution. The solution was stirred overnight at room temperature. After stirring, the disappearance of p-phenyl diisothiocyanate was observed by TLC (7/3 DCM/MeOH). The solvent was evaporated and the mixture was dissolved in a minimum of MeOH. The precipitation of compound 7 was performed by adding diethyl ether. The precipitate was washed 3 times with diethyl ether to afford 23 mg of a white powder. The precipitate was washed 3 times with diethyl ether to afford 23 mg of a white powder. This mixture was directly used for the synthesis of compound (I)-c without further purification.

*Compound **(I)-c**.* Compound **7** (23 mg, 1.77×10⁻⁵ mol, 1 eq.) was suspended in 2 mL of a mixture of water and THF 1/1. LiOH (7.3 mg, 3.04×10⁻⁴mol, 17 eq.) was added in the solution. Compound **2** dissolves thanks to LiOH. The solution was stirred overnight at room temperature. The disappearance of compound **2** was checked by HPLC (H₂O 0.1 % TFA, ACN 0.1 % TFA). The solvent was evaporated and the mixture was purified by preparative HPLC (100 % H₂O 0.1 % TFA to 8/2 H₂O 0.1 % TFA/ACN 0.1 % TFA) to afford 24 mg (white powder) of compound **3** which was isolated in a form of a TFA salt in a 49% yield over 2 steps.
¹H NMR (500 MHz, MeOD) δ = 8.82 (m, 4H, Ha +Ha'), 7.90 (tdd, *J*=7.8, 3.1, 1.8, 4H, Hc + Hc'), 7.77 (m, 4H, Hb + Hb'), 7.55 - 7.43 (m, 4H, Hd + Hd'), 7.32 (s, 4H, He), 5.57 (s, 2H, H2/H4), 5.59 (s, 2H, H2/H4), 4.42 (s, 2H, H9), 3.78 - 3.61 (m, 4H, H14), 3.52 (dd, *J*=12.4, 1.8, 2H, H6 + H8), 3.40 - 3.33 (m, 2H, H10), 3.15 - 3.01 (m, 6H, H6 + H8), 2.37 (m, 2H, H11/11'), 2.31 (s, 6H, H15), 1.94 - 1.74 (m, 6H, H13 + H11/11'), 1.64 (m, 2H, H12/12'), 1.57 (m, 2H, H12/12').
¹³C NMR (126 MHz, Methanol-*d*₄) δ = 181.23 (C_{quat}), 172.45 (C_{quat}), 170.42 (C_{quat}), 170.24 (C_{quat}), 160.81 (C_{quat}), 151.83 (C_{quat}), 151.76 (C_{quat}), 149.57 (CHₐᵣ), 149.17 (CHₐᵣ), 137.43 (CHₐᵣ), 137.30 (CHₐᵣ), 127.58 (CHₐᵣ), 127.25 (CHₐᵣ), 125.38 (CHₐᵣ)), 124.60 (CHₐᵣ), 124.57 (CHₐᵣ), 72.64 (CH), 67.11 (CH), 66.54 (CH), 65.99 (CH), 55.02 (CH₂), 51.90 (C_{quat}), 51.36 (C_{quatz}), 48.92 (CH₂), 44.26 (CH₂), 44.34 (CH₂), 42.04 (CH₃), 28.89 (CH₂), 28.64 (CH₂), 24.04 (CH₂).
MS (ESI+, H₂O): *m*/*z* 624.24 ([M+2H]²⁺, 40%), 631.24 ([M+2Li]²⁺, 100%), 1247.4 ([M+H]⁺, 1%), 631.24 ([M+2Li]⁺, 3%).

### Example 5: synthesis of compound (I-c)

Compound **(I-g)** was prepared according to the following scheme 5:

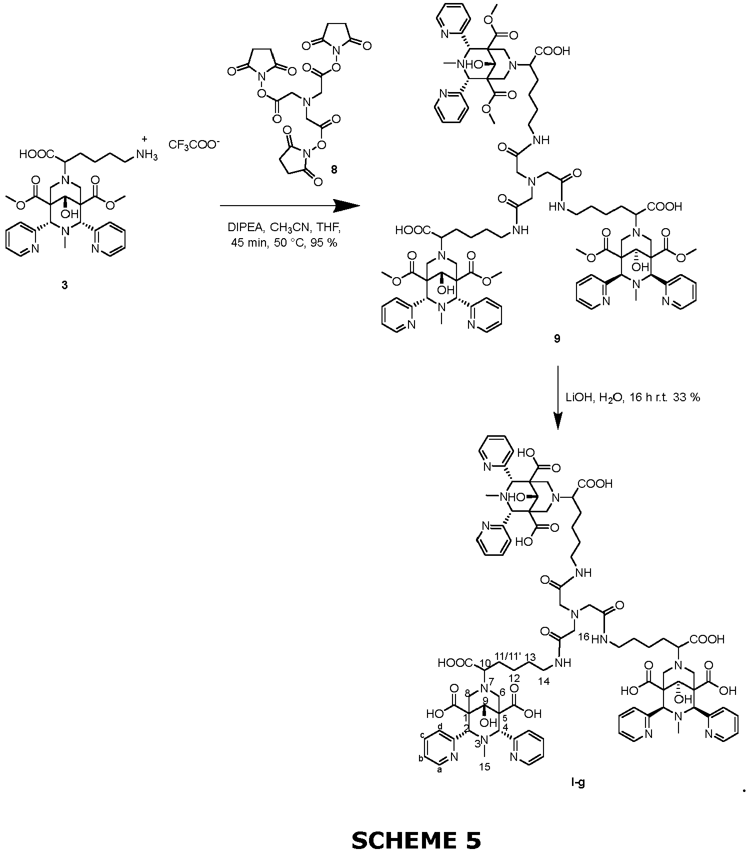

*Compound* **9:** Compound **3** (124, 0.16 mmol) and DIPEA (140 µl, 0.8 mmol) are dissolved in 15 ml of DMF. Compound **8** (22 mg, 0.046 mmol) is added, and the mixture is stirred for 45 min at 50 °C. The solvent is removed under vacuum and the crude product is purified by FPLC (C₁₈, H₂O/ACN, 0.1 % TFA) to give compound **9** with 95 % yield.
¹H NMR (400 MHz, MeOD): *δ* 8.78 (m, 6H, Ha); 7.91 (m, 6H, Hc); 7.73(m, 6H, Hd); 7.50 (m, 6H, Hb); 5.60 (s, 3H, H2/H4); 5.58 (s, 3H, H2/H4); 4.38 (s, 3H, H9); 3.82(s, 6H, H16); 3.71 (s, 9H, OCH₃); 3.68 (s, 9H, OCH₃); 3.54 (m, 3H, H6/H8); 3.36 (m, 9H, H10 + H14); 3.10 (m, 9H, H6/H8); 2.29 (m, 12H, H11/11' + H15); 1.86 (m, 3H, H11/11'); 1.73 (m, 6H, H13); 1.54 (m, 6H, H12).

*Compound (**I-g**):* Compound **9** (42 mg, 0.023 mmol) is dissolved in 6 ml of water. Lithium hydroxide (13 mg, 0.53 mmol) is added, and the mixture is stirred at room temperature overnight. The solvent is removed under vacuum and the crude product is purified by FPLC (C₁₈, H₂O/ACN, 0.1 % TFA) to give compound **(I-g)** (15 mg) with 33 % yield.
¹H NMR (400 MHz, MeOD): *δ* 8.76 (m, 6H, Ha); 7.88 (m, 6H, Hc); 7.76 (m, 6H, Hd); 7.49 (m, 6H, Hb); 5.57 (s, 3H, H2/H4); 5.55 (s, 3H, H2/H4); 4.38 (s, 3H, H9); 3.78 (s, 6H, H16); 3.45 (m, 3H, H6/H8); 3.35 (m, 9H, H10 + H14); 3.03 (m, 9H, H6/H8); 2.29 (m, 12H, H11/11' + H15); 1.75 (m, 6H, H13); 1.53 (m, 6H, H12).

### Example 6: synthesis of a complex of manganese (II) [(I-A)] with bispidine-based metal chelating ligands (I-a)

A complex **(I-A)** of manganese (II) with chelating bispidine-based ligand **(I-a)** of example 2 was prepared as follows:
409 µl of a solution of **(I-a)** at 1.5 mM is mixed to 50 µL of MnCl₂ at 98.3 mM solution. MilliQ water is added to reach a volume of 1.5 ml After 30 min at 60 °C the pH was increased from 4 to 7 and the mixture was heated at 60 °C overnight. The end of the reaction is monitored by HPLC. At the end of the reaction, the solvent is removed under vacuum and the crude is purified on HPLC preparative (C₁₈, ACN/H₂O, 0.1 % TFA). A solution of 800 µl of **(I-A)** at 1.59 mM (determined by ICP-AES) is finally obtained.

The same preparation method was used to obtain a complex **(I-B)** of manganese (II) with compound **4,** which is not part of the invention.

Table 1 below shows the properties of complex (I-A), and for comparison of complex **(I-B).** Longitudinal relaxivity r₁ is exprimed per millimolar concentration of complex. Longitudinal relaxivity r_{1,Mn} is exprimed per millimolar concentration of Mn(II).

**TABLE 1**

| **Compound** | **Longitudinal relaxivity in water (r₁; mM⁻¹/s) at 25°C, pH = 7.4, and 60 MHz** | **Longitudinal relaxivity in water (r_{1,Mn}; mM⁻¹/s) at 25°C, pH = 7.4, and 60 MHz** |
|---|---|---|
| **(I-A)** | 10.98 | 5.49 |
| **(I-B)^{∗}** | 4.03 | 4.03 |

| | | |
|---|---|---|
| * Not part of the invention | | |

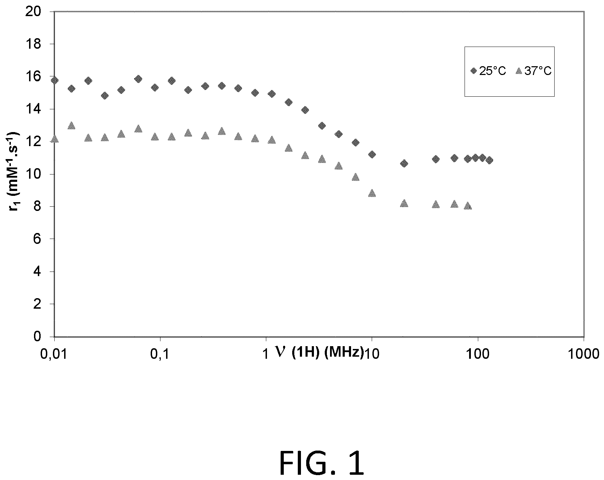

Figure 1 represents the NMRD profile (longitudinal relaxivity r₁ as a function of proton larmor frequency in mM⁻¹.s⁻¹ as a function of the frequency in MHz) of complex **(I-A)** in water at 25°C (curve with diamonds) and at 37°C (curve with triangles).

The relaxivity at 60MHz is measured with a device commercialized under the brand name WP80 NMR by the firm Brucker according to the following protocol. A millimolar aqueous solution of complex was prepared with Milli-Q water (ρ < 18MΩ). The concentration of Mn²⁺-containing samples was checked by ICP-OES and/or NMR by using the bulk magnetic susceptibility. The absence of free Mn²⁺ was checked by the Xylenol orange test. For the relaxivity measurement, the temperature was monitored by a VTC91 temperature control unit and maintained by a gas flow. The temperature was determined by previous calibration with a Pt resistance temperature probe.

## Claims

1. A bispidine-based metal chelating ligand responding to the following formula (I):
L-(R)ₙ (I)
wherein n represents an integer such as n ≥ 2, and R groups represent, independently from each other,
• a group R¹-(T¹)ᵣ-CH₂^{∗}-, in which T¹ represents a C₁-C₁₀ alkylene group, and r represents an integer 0 or 1, and/or
• a group responding to the following formula (II): 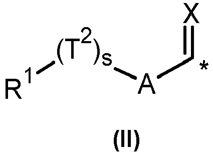 in which:
- T² represents a C₁-C₁₀ alkylene group,
- A represents -CH₂- or -NH-,
- X represents an oxygen atom, a sulfur atom, or a NH group,
- s represents an integer 0 or 1 when A represents -CH₂- and s represents an integer 1 when A represents -NH-; and
wherein ^{∗} indicates the point of attachment of said group R with L,
wherein R¹ is a bispidol group of the following formula (III): 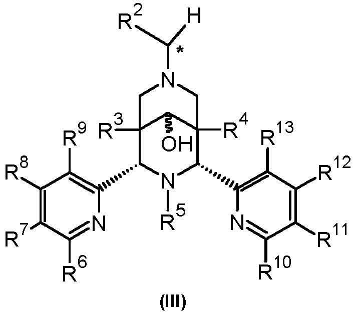 in which:
- R² represents a PO₃H₂ group or a CO₂H group,
- R³ and R⁴, which may be identical or different, represent a CO₂H group or a CH₂OH group,
- R⁵ represents a hydrogen atom, an alkyl group, or a group of formula (IV): -T³-CO₂H (IV) where T³ represents a C₁-C₅ alkylene group,
- R⁶, R⁷, R⁸, R⁹, R¹⁰, R¹¹, R¹², and R¹³, independently from each other, represent a hydrogen atom, an OH group, an ether group OR¹⁴ where R¹⁴ is an alkyl group, a CO₂H group, or a CONHR¹⁵ group, where R¹⁵ represents an alkyl group,
- when r, respectively s, represents an integer 1, ^{∗} indicates the point of attachment of said group R¹ with T¹, respectively with T², and when r, respectively s, represents an integer 0, ^{∗} indicates the point of attachment of said group R¹ with -CH₂-, respectively with -A-,
wherein L is at least a divalent linker selected from:
• a single bond,
• a guadinine group,
• an urea group,
• a thiourea group,
• a C₅-C₁₈ aryl group,
• a C₂-C₁₈ heteroaryl group,
• a C₂-C₁₀ alkyl group,
• a group responding to formula (V):
^{∗}NH-D(NH^{∗})ₜ-NH^{∗} (V)
in which D is selected from an aryl group, an heteroaryl group, an alkyl group, a 3-cyclobutene-1,2-dione group, a polyethylene group, and a polyaminoacid group, and t is an integer ranging from 0 to 6,
• a group responding to the following formula (VI):
^{∗}NH-(CR¹⁶R¹⁷)ₘ₁-[NR²⁰-(CR¹⁸R¹⁹)_{q1}]ₚ₁-NH^{∗} (VI)
in which:
- R¹⁶ and R¹⁷ represent, independently from each other, independently at each occurrence m1, a hydrogen atom or a C₁-C₅ alkyl group,
- m1 is an integer ranging from 2 to 5,
- R¹⁸ and R¹⁹ represent, independently from each other, independently at each occurrence q1, independently at each occurrence p1, a hydrogen atom or a C₁-C₅ alkyl group,
- R²⁰ represents, independently at each occurrence p1, a hydrogen atom, a C₁-C₅ alkyl group, a group -T⁴-NH^{∗}- in which T⁴ represents a C₂-C₅ alkylene group, or a single bond*,
- q1 represents, independently at each occurrence p1, an integer ranging from 2 to 5, and
- p1 is an integer ranging from 0 to 4,
• a group responding to the following formula (VII):
^{*}E-NH-(CR¹⁶R¹⁷)ₘ₁-[NR²¹-(CR¹⁸R¹⁹)_{q1}]ₚ₁-NH-E^{∗} (VII)
in which:
- R¹⁶, R¹⁷, R¹⁸, and R¹⁹ are as defined above,
- m1, q1, and p1 are as defined above,
- R²¹ represents, independently at each occurrence p1, a hydrogen atom, a C₁-C₅ alkyl group, a group -T⁵-NH-E^{∗} in which T⁵ represents a C₂-C₅ alkylene group, or a single bond*,
- E is a 3-amino-, or 4-amino-3-cyclobutene-1,2-dione group, or a C₁-C₅ alkyl group,
• a group responding to the following formula (VIII):
^{∗}(CR²²R²³)ₘ₂-NR²⁸-[(CR²⁴R²⁵)_{q2}-NR²⁹]ₚ₂-(CR²⁶R²⁷)ₘ₃^{∗} (VIII)
in which:
- R²² and R²³ represent, independently from each other, independently at each occurrence m2, a hydrogen atom or a C₁-C₅ alkyl group,
- m2 is an integer ranging from 1 to 5,
- R²⁴ and R²⁵ represent, independently from each other, independently at each occurrence q2, independently at each occurrence p2, a hydrogen atom or a C₁-C₅ alkyl group,
- R²⁶ and R²⁷ represent, independently from each other, independently at each occurrence m3, a hydrogen atom or a C₁-C₅ alkyl group,
- m3 is an integer ranging from 1 to 5,
- R²⁸ represents a hydrogen atom, a C₁-C₅ alkyl group, a C₁-C₅ alkylene group^{∗}, or a single bond^{∗},
- R²⁹ represents, independently at each occurrence p2, a hydrogen atom, a C₁-C₅ alkyl group, a C₁-C₅ alkylene group^{∗}, a single bond^{∗}, or a - [(CR²⁴R²⁵)_{q2}-NR²⁹]ₚ₂-(CR²⁶R²⁷)ₘ₃^{∗} group,
- q2 represents, independently at each occurrence p2, an integer ranging from 2 to 5; and
- p2 is an integer ranging from 0 to 4,
• a group responding to the following formula (IX):
^{∗}(CR³⁰R³¹)ₘ₄-NR³⁴-[(CH₂-CH₂-O)_{q4}-CH₂-CH₂-NR³⁵]p₄-(CR³²R³³)ₘ₅^{∗} (IX)
in which:
- R³⁰ and R³¹ represent, independently from each other, independently at each occurrence m4, a hydrogen atom or a C₁-C₅ alkyl group,
- m4 is an integer ranging from 1 to 5,
- R³² and R³³ represent, independently from each other, independently at each occurrence m5, a hydrogen atom or a C₁-C₅ alkyl group,
- m5 is an integer ranging from 1 to 5,
- R³⁴ represents a hydrogen atom, a C₁-C₅ alkyl group, a C₁-C₅ alkylene group^{∗}, or a single bond^{∗},
- R³⁵ represents, independently at each occurrence p4, a hydrogen atom, a C₁-C₅ alkyl group, a C₁-C₅ alkylene group^{∗}, or a single bond^{∗},
- q4 represents, independently at each occurrence p4, an integer ranging from 1 to 5, and
- p4 is an integer ranging from 0 to 4,
wherein * in formulae (V) to (IX) indicates the possible points of attachment of said linker L with groups R,
• a polyazacycloalkane saturated group in which at least two -N-functions represent points of attachment of said linker L with groups R,
• a polyaminoacid group in which at least two functions represent points of attachment of said linker L with groups R, said function being a carbonyl function (derived from the carboxylic acid function), a thiol function, or an amine function,
• a polyamidoamine group in which at least two -NH- functions represent points of attachment of said linker L with groups R.

2. The ligand according to claim 1, wherein at least two R groups represent a group R¹-T¹-CH₂^{∗}-, in which T¹ represents a C₁-C₄ alkylene group.

3. The ligand according to claim 1 or claim 2, wherein at least two R groups represent a group responding to said following formula (II), where A represents -NH-, and X represents an oxygen atom, a sulfur atom, or a NH group, or A represents -CH₂- and X represents an oxygen atom.

4. The ligand according to any one of the preceding claims, wherein R³ and R⁴ represent CO₂H groups.

5. The ligand according to any one of the preceding claims, wherein R² represents a CO₂H group.

6. The ligand according to any one of the preceding claims, wherein L is a polyazacycloalkane saturated group selected from cyclen, cyclam, and derivatives thereof.

7. The ligand according to any one of the preceding claims, wherein R⁵ is a linear C₁-C₅ alkyl group.

8. The ligand according to any one of the preceding claims, wherein at least R⁷ and R¹¹, and/or R⁹ and R¹³ represent hydrogen atoms, and preferably R⁶, R⁷, R⁸, R⁹, R¹⁰, R¹¹, R¹², and R¹³ groups represent hydrogen atoms.

9. The ligand according to any one of the preceding claims, wherein L responds to any one of the following formula (V): 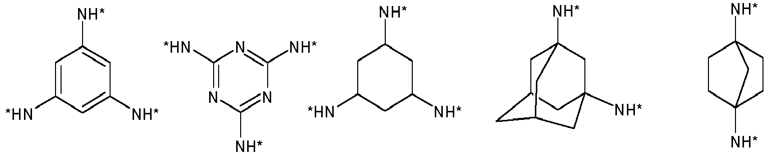 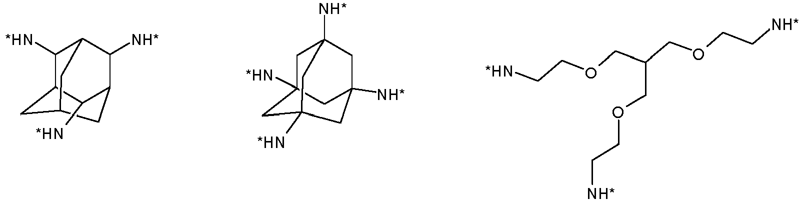 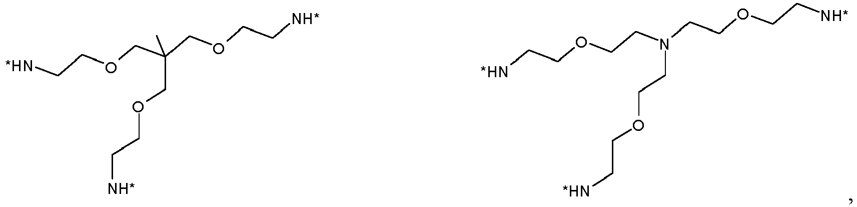 wherein * represent the possible points of attachment of the linker L to the group R.

10. The ligand according to any one of the preceding claims, wherein L responds to the any one of the following formula (VI): 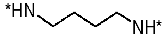 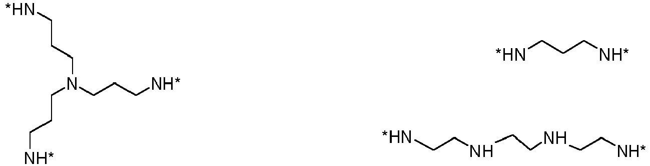 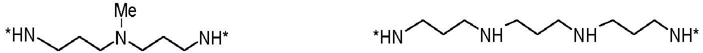 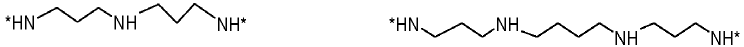 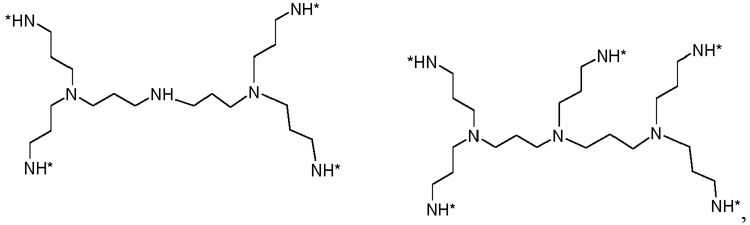 wherein * represent the possible points of attachment of the linker L to the group R.

11. The ligand according to any one of the preceding claims, wherein L responds to the following formula (VII): 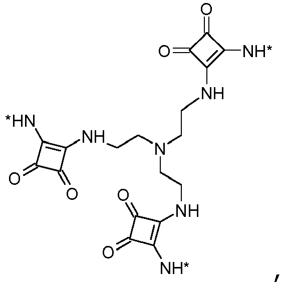 wherein * represent the possible points of attachment of said linker L to the group R.

12. The ligand according to any one of the preceding claims, wherein L responds to any one the following formula (VIII): 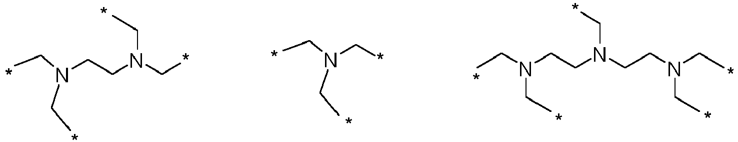 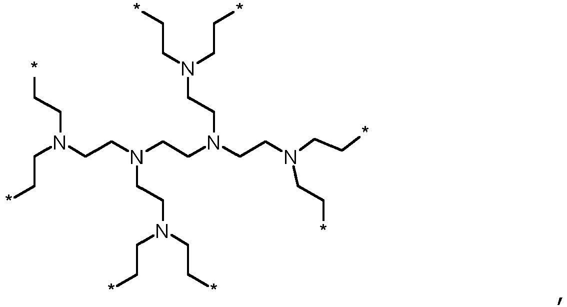 wherein * represent the possible points of attachment of the linker L to the group R.

13. The ligand according to any one of the preceding claims, wherein said ligand is selected from the following formulae (I-a) to (I-i):

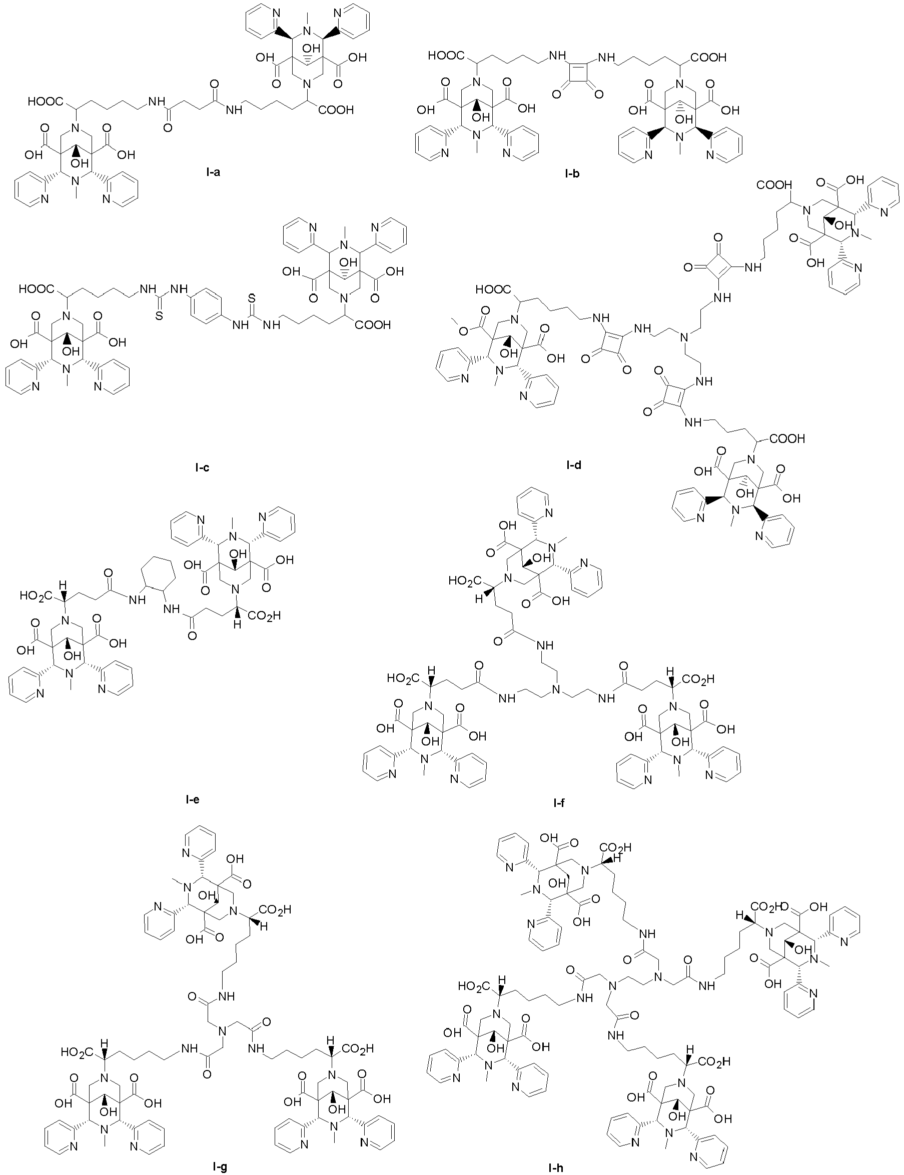

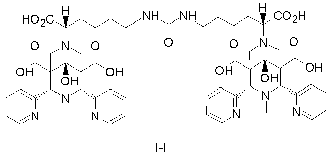

14. A complex of a metal M, wherein said complex comprises several metal ions of said metal M complexed with a bispidine-based metal chelating ligand (I) as defined in any one of the preceding claims, said metal M being selected from copper, manganese, gallium, cobalt, zinc, nickel, and iron.

15. Use of a bispidine-based metal chelating ligand (I) as defined in any one of claims 1 to 13, or of a complex as defined in claim 14, in the field of medical imaging or therapy, and more specifically as MRI contrast agents and/or nuclear imaging agents for PET or SPECT.
